# EUROPEAN PATENT APPLICATION

(11) **EP 4 670 736 A1**
(43) Date of publication of application: **31.12.2025**
(21) Application number: 24760398.8
(22) Date of filing: 21.02.2024
(51) Int. Cl.: A61K 45/00, A61K 31/194, A61K 33/10, A61P 25/02, A61P 35/00, A61P 43/00

(54) **PHARMACEUTICAL FOR TREATING OR PREVENTING CHEMOTHERAPY-INDUCED PERIPHERAL NEUROPATHY**

(30) Priority: 24.02.2023 JP 2023027461; 30.10.2023 JP 2023185444
(71) Applicant: Nippon Chemiphar Co., Ltd., Tokyo 101-0032 (JP); National University Corporation University of Toyama, Toyama-shi, Toyama 930-8555 (JP)
(72) Inventor: UTA, Daisuke, Toyama-shi, Toyama 930-0194 (JP); HASHIMOTO, Satoshi, Tokyo 101-0032 (JP); NAKAMURA, Hideki, Misato-shi, Saitama 341-0005 (JP); KUNIGAMI, Toshihiro, Misato-shi, Saitama 341-0005 (JP)
(74) Representative: Kraus & Lederer PartGmbB
(86) International application number: PCT/JP2024/006184
(87) International publication number: WO 2024/177100

(57) **Abstract**

Provided is a pharmaceutical composition for treating or preventing peripheral neuropathy induced by a chemotherapy drug, the pharmaceutical composition containing an alkalinizing agent. Administration of this pharmaceutical composition enables the treatment or prevention of peripheral neuropathy induced by a chemotherapy drug, particularly a cancer chemotherapy drug. Also provided is an anticancer pharmaceutical composition which contains an alkalinizing agent and a cancer chemotherapy drug, and exhibits suppressed expression of peripheral neuropathy. Administration of this pharmaceutical composition enables cancer treatment with suppressed expression of peripheral neuropathy.

## Description

### Technical Field

The present invention relates to a novel pharmaceutical composition containing an alkalinizing agent. More specifically, the present invention relates to a pharmaceutical composition for treating or preventing peripheral neuropathy induced by chemotherapy drugs, the composition containing an alkalinizing agent.

Priority is claimed on Japanese Patent Application No. 2023-027461, filed February 24, 2023, and Japanese Patent Application No. 2023-185444, filed October 30, 2023, the contents of which are incorporated herein by reference.

### Background Art

Cancer chemotherapy can, for example, sometimes cause hypoesthesia or sensory disorders in the limbs. These are symptoms caused by peripheral neuropathy, and tend to manifest most strongly at the periphery of the limbs. Symptoms of chemotherapy-induced peripheral neuropathy (sometimes abbreviated as CIPN in this description) vary from unpleasant dysesthesia to sometimes hindering daily life, and in severe cases may sometimes prevent walking or dressing. Transient CIPN in which recovery occurs rapidly when administration of the anticancer drug is stopped tends to be unusual, with improvement often requiring months or years, and even lifelong retention of some form of damage is not rare.

Although CIPN is an adverse event that has long been known, many facets of CIPN including the mechanism remain unclear, and no effective treatment method has yet been established. A variety of drugs are currently used for treating CIPN, but none yields a totally satisfactory effect. CIPN is an important adverse event hindering sustained chemotherapy, and preventing or treating CIPN would not only improve the results of cancer treatment, but could also contribute to an improvement in the patient QOL, but current countermeasures rely on halting the administration of the chemotherapeutic agent causing the CIPN (Non-Patent Document 1).

Symptomatologic classification of CIPN is expressed using terms such as sensory nerve damage, numbness, tingling, or pain or the like. Further, severe pain is sometimes referred to as shooting pain or burning pain. In terms of the relationships between these symptoms, numbness and tingling is not necessarily accompanied by severe pain, but patients with severe pain almost all report having numbness or tingling. CIPN numbness presents as muscular atrophy predominantly at the limb extremities, reduced muscle strength, or flaccid paralysis. Further, a deterioration or loss of limb tendon reflex has also been recognized, with these symptoms becoming more marked closer to the limb extremities. CIPN autonomic neuropathy can impact blood pressure, intestine motility and involuntary muscles, with recognized symptoms including urinary disturbance, sweating disorder, orthostatic hypotension, constipation, or paralytic ileus.

In order to prevent CIPN, calcium or magnesium administration is sometimes administered with the aim of achieving a neurotoxicity preventive effect, but there is no clear evidence of that effect. Gosha-jinki-gan, vitamin B12 formulations, non-steroidal anti-inflammatory analgesics, pregabalin, gabapentin or opioids are sometimes administered for the treatment of numbness or pain, but none of these exhibits clear efficacy, and there is no evidence to recommend their administration (Non-Patent Document 1). Duloxetine is the only drug listed in the guidelines for which there is moderate evidence of efficacy (Non-Patent Document 2).

However, it is known that duloxetine frequently requires administration to be halted soon after starting administration due to adverse events (Non-Patent Document 3).

On the other hand, alkalinizing agents including some citric acid formulations or sodium bicarbonate are known to be effective against the representative lifestyle related disease of hyperuricemia or gout. Further, administration of an alkalinizing agent is known to suppress the production of urinary stones by making acidic urine more alkaline. Furthermore, it is known that administration of an alkalinizing agent to early-stage CKD patients suppresses the progression of kidney damage, and reduces the concentration of uremic substances in the blood (Patent Documents 1 and 2). Moreover, citric acid formulations are known to suppress renal fibrosis in diabetic nephropathy (Patent Document 3).

However, the effectiveness of alkalinizing agents in the prevention or treatment of chemotherapy-induced peripheral neuropathy is unknown.

### Citation List

### Patent Documents

Patent Document 1
   WO 2018/193648
Patent Document 2
   WO 2018/193752
Patent Document 3
   WO 2020/080451

### Non Patent Documents

Non Patent Document 1
   Guide to the Management of Peripheral Neuropathy accompanying Cancer Drug Therapy
Non Patent Document 2
   Guidelines for the Pharmacologic Management of Neuropathic Pain, Second Edition
Non Patent Document 3
   Investigation of Risk Factors for Duloxetine-Induced Adverse Events in Cancer Patients

### Summary of Invention

### Problems to be solved by Invention

One object of the present invention is to provide a pharmaceutical that is useful for treating or preventing peripheral neuropathy induced by chemotherapy drugs.

Another object of the present invention is to provide an anticancer combination formulation which is composed of an alkalinizing agent and a cancer chemotherapy drug and exhibits suppressed expression of peripheral neuropathy.

Yet another object of the present invention is to provide an anticancer pharmaceutical composition which contains an alkalinizing agent and a cancer chemotherapy drug, and exhibits suppressed expression of peripheral neuropathy.

Yet another object of the present invention is to provide an anticancer pharmaceutical kit which contains an alkalinizing agent and a cancer chemotherapy drug, and exhibits suppressed expression of peripheral neuropathy.

Yet another object of the present invention is to provide a method for treating or preventing peripheral neuropathy induced by chemotherapy drugs.

Yet another object of the present invention is to provide a cancer treatment method that exhibits suppressed expression of peripheral neuropathy.

### Means to Solve the Problems

As a result of intensive investigation aimed at achieving the above objects, the inventors of the present invention discovered that a specific alkalinizing agent was useful in treating or preventing peripheral neuropathy induced by chemotherapy drugs, enabling them to complete the present invention.

In other words, the present invention has the following aspects.
(1) A pharmaceutical composition for treating or preventing peripheral neuropathy induced by a chemotherapy drug, the composition containing an alkalinizing agent.
(2) The pharmaceutical composition according to (1), wherein the peripheral neuropathy induced by a chemotherapy drug is pain, sensory disturbance, mobility impairment, autonomic neuropathy, or a complication thereof attributable to peripheral neuropathy.
(3) The pharmaceutical composition according to (1) or (2), wherein the peripheral neuropathy induced by a chemotherapy drug is peripheral neuropathic pain.
(4) The pharmaceutical composition according to (3), wherein the peripheral neuropathic pain is shooting pain, burning pain, pain (excluding shooting pain and burning pain), or tingling.
(5) The pharmaceutical composition according to (1) or (2), wherein the peripheral neuropathy induced by a chemotherapy drug is a sensory disturbance attributable to peripheral neuropathy.
(6) The pharmaceutical composition according to (5), wherein the sensory disturbance attributable to peripheral neuropathy is hypoesthesia, hyperesthesia or a sensory disorder.
(7) The pharmaceutical composition according to (6), wherein the hyperesthesia is allodynia, and the sensory disorder is dysesthesia or paresthesia.
(8) The pharmaceutical composition according to (1) or (2), wherein the peripheral neuropathy induced by a chemotherapy drug is autonomic neuropathy attributable to peripheral neuropathy.
(9) The pharmaceutical composition according to (8), wherein a symptom caused by the autonomic neuropathy is urinary disturbance, sweating disorder, orthostatic hypotension, constipation, or paralytic ileus.
(10) The pharmaceutical composition according to any one of (1) to (9), wherein the peripheral neuropathy induced by a chemotherapy drug is peripheral neuropathy having symptoms that manifest in the four limbs.
(11) The pharmaceutical composition according to any one of (1) to (10), wherein the alkalinizing agent is a pharmaceutically acceptable salt of citric acid or a hydrate thereof, or a mixture thereof.
(12) The pharmaceutical composition according to any one of (1) to (11), wherein the alkalinizing agent is sodium citrate, potassium citrate, a hydrate of sodium citrate or potassium citrate, or a mixture thereof.
(13) The pharmaceutical composition according to any one of (1) to (12), wherein the alkalinizing agent contains a mixture of sodium citrate or a hydrate thereof, and potassium citrate or a hydrate thereof.
(14) The pharmaceutical composition according to any one of (1) to (13), wherein the alkalinizing agent is sodium citrate or a hydrate thereof.
(15) The pharmaceutical composition according to any one of (1) to (14), wherein the pharmaceutical composition is a tablet.
(16) The pharmaceutical composition according to any one of (1) to (10), wherein the alkalinizing agent is sodium bicarbonate.
(17) The pharmaceutical composition according to any one of (1) to (16), wherein the chemotherapy drug is a cancer chemotherapy drug.
(18) The pharmaceutical composition according to (17), wherein the cancer chemotherapy drug is a cytotoxic anticancer drug, molecular targeted anticancer drug, immunotherapy drug, or other form of anticancer drug.
(19) The pharmaceutical composition according to (18), wherein the cytotoxic anticancer drug is at least one anticancer drug selected from the group consisting of paclitaxel, docetaxel, cabazitaxel, vinorelbine, vincristine, vinblastine, vindesine, eribulin, oxaliplatin, carboplatin, cisplatin and nelarabine.
(20) The pharmaceutical composition according to (18), wherein the molecular targeted anticancer drug is at least one anticancer drug selected from the group consisting of bortezomib, ixazomib, romidepsin, gilteritinib, trastuzumab emtansine, brentuximab vedotin, obinutuzumab, blinatumomab, lorlatinib, entrectinib, encorafenib, binimetinib and pemigatinib.
(21) The pharmaceutical composition according to (18), wherein the immunotherapy drug is at least one anticancer drug selected from the group consisting of nivolumab, ipilimumab, pembrolizumab, atezolizumab and avelumab.
(22) The pharmaceutical composition according to (18), wherein the other form of anticancer drug is at least one anticancer drug selected from the group consisting of cytarabine, ifosfamide, nedaplatin, thalidomide, lenalidomide and pomalidomide.
(23) The pharmaceutical composition according to (17), wherein the cancer chemotherapy drug is at least one anticancer drug selected from the group consisting of paclitaxel, vincristine, oxaliplatin and bortezomib.
(24) The pharmaceutical composition according to (23), wherein the cancer chemotherapy drug is paclitaxel or oxaliplatin.
(25) A combination formulation for simultaneous, separate, or sequential administration in a cancer treatment, wherein
   the combination formulation includes at least two separate formulations composed of an alkalinizing agent and a cancer chemotherapy drug, and exhibits suppressed expression of peripheral neuropathy.
(26) An anticancer pharmaceutical composition that exhibits suppressed expression of peripheral neuropathy, the composition containing an alkalinizing agent and a cancer chemotherapy drug.
(27) An anticancer pharmaceutical kit containing an alkalinizing agent in a first compartment and a cancer chemotherapy drug in a second compartment, wherein the pharmaceutical kit exhibits suppressed expression of peripheral neuropathy.

### Effects of Invention

The pharmaceutical composition, combination formulation and pharmaceutical kit provided by the present invention, enable the treatment or prevention of peripheral neuropathy induced by chemotherapy drugs. Furthermore, the pharmaceutical composition, combination formulation and pharmaceutical kit provided by the present invention enable cancer treatments to be conducted with good suppression of peripheral neuropathy induced by chemotherapy drugs, and particularly cancer chemotherapy drugs.

### Brief Description of the Drawings

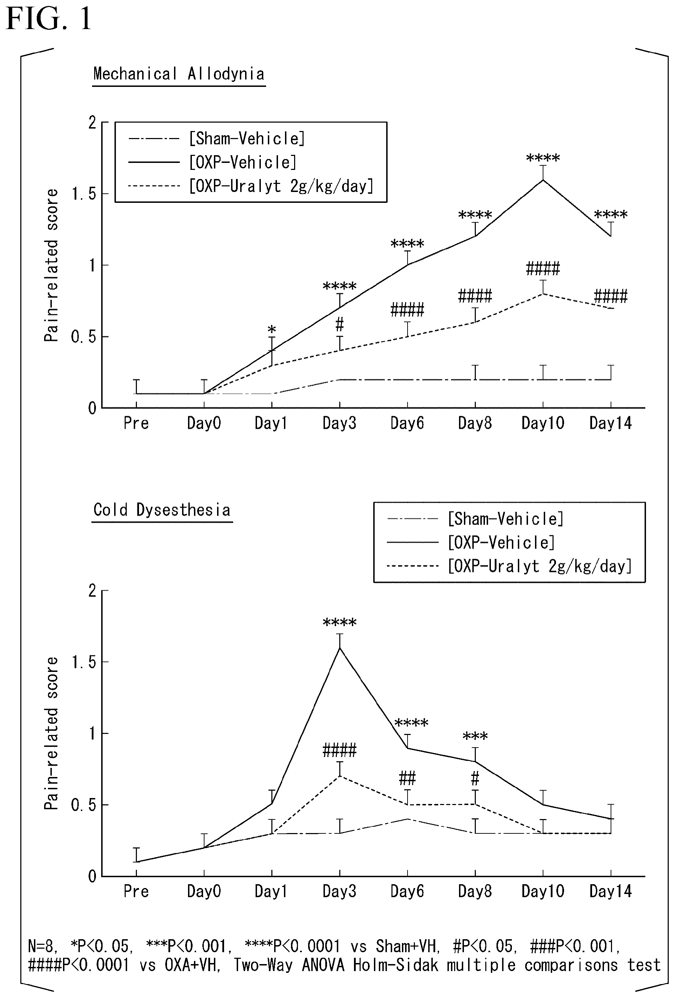

FIG. 1 is a diagram illustrating the effect of an alkalinizing agent in an oxaliplatin-induced mouse neuropathic pain model, and shows the relationship between the number of days elapsed from the start of oxaliplatin administration and the pain-related score for mechanical allodynia and cold dysesthesia.

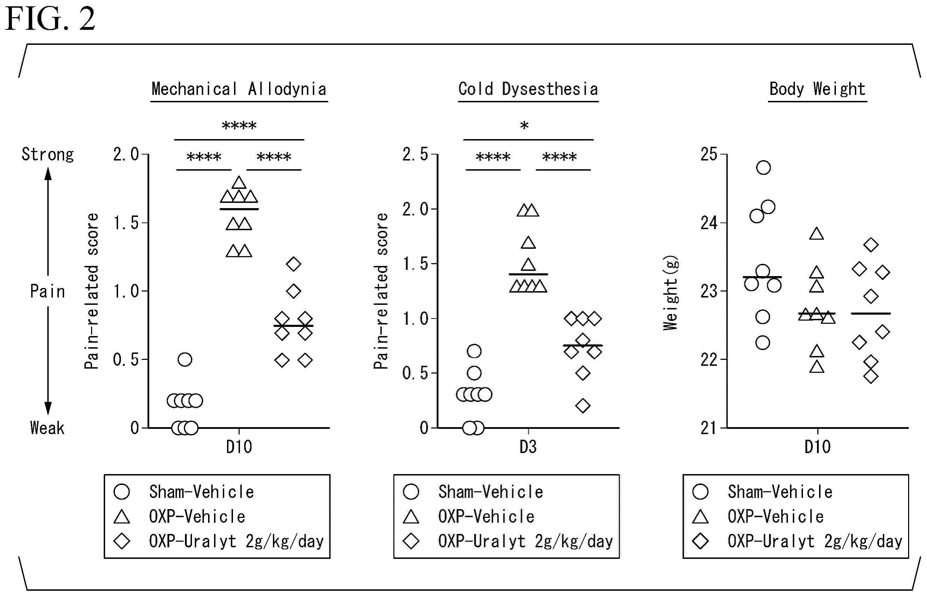

FIG. 2 is a diagram illustrating the effect of an alkalinizing agent in an oxaliplatin-induced mouse neuropathic pain model, and shows the pain-related score for each test group in relation to mechanical allodynia on day 10 from the start of oxaliplatin administration, the pain-related score for each test group in relation to cold dysesthesia on day 3 from the start of oxaliplatin administration, and the body weight of each test group on day 10 from the start of oxaliplatin administration.

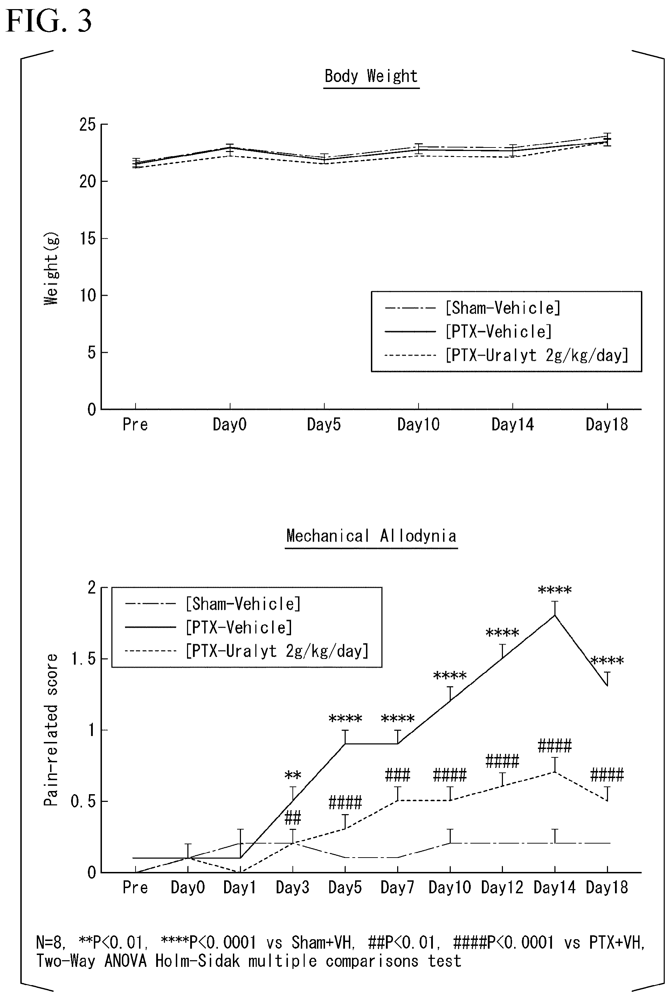

FIG. 3 is a diagram illustrating the effect of an alkalinizing agent in a paclitaxel-induced mouse neuropathic pain model, and shows the relationship between the number of days elapsed from the start of paclitaxel administration and the body weight, and the relationship between the number of days elapsed from the start of paclitaxel administration and the pain-related score in relation to mechanical allodynia.

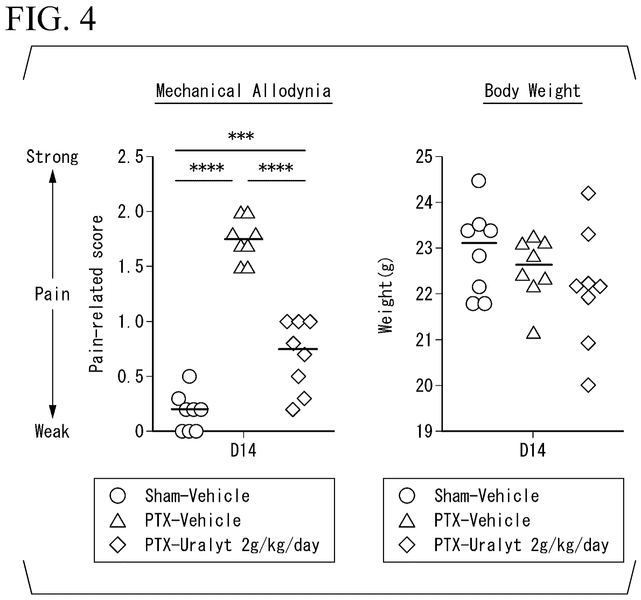

FIG. 4 is a diagram illustrating the effect of an alkalinizing agent in a paclitaxel-induced mouse neuropathic pain model, and shows the pain-related score for each test group in relation to mechanical allodynia on day 14 from the start of paclitaxel administration, and the body weight of each test group on day 14 from the start of paclitaxel administration.

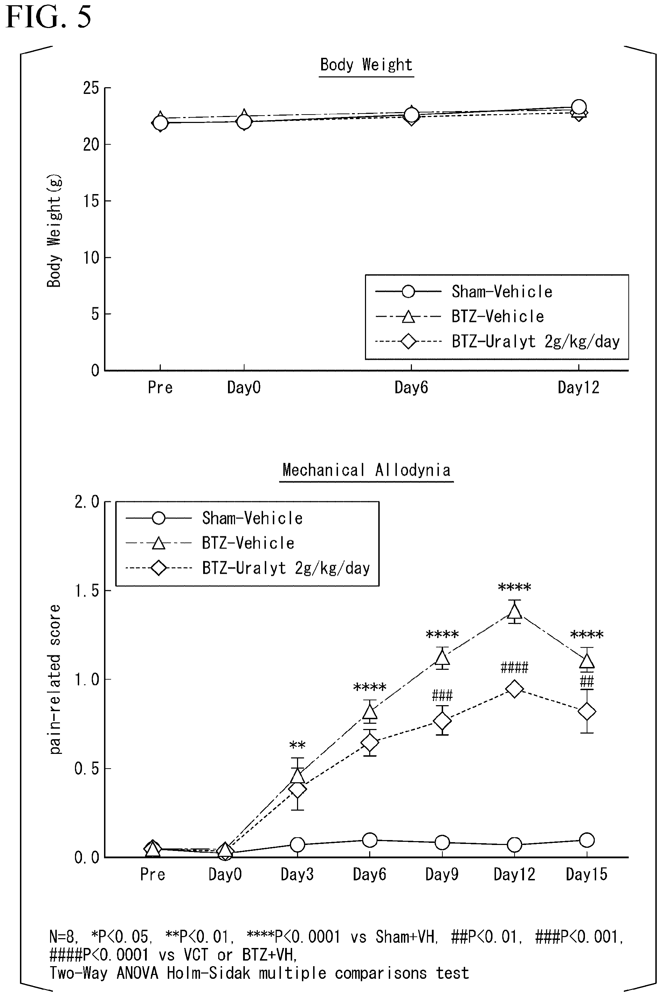

FIG. 5 is a diagram illustrating the effect of an alkalinizing agent in a bortezomib-induced mouse neuropathic pain model, and shows the relationship between the number of days elapsed from the start of bortezomib administration and the body weight, and the relationship between the number of days elapsed from the start of bortezomib administration and the pain-related score in relation to mechanical allodynia.

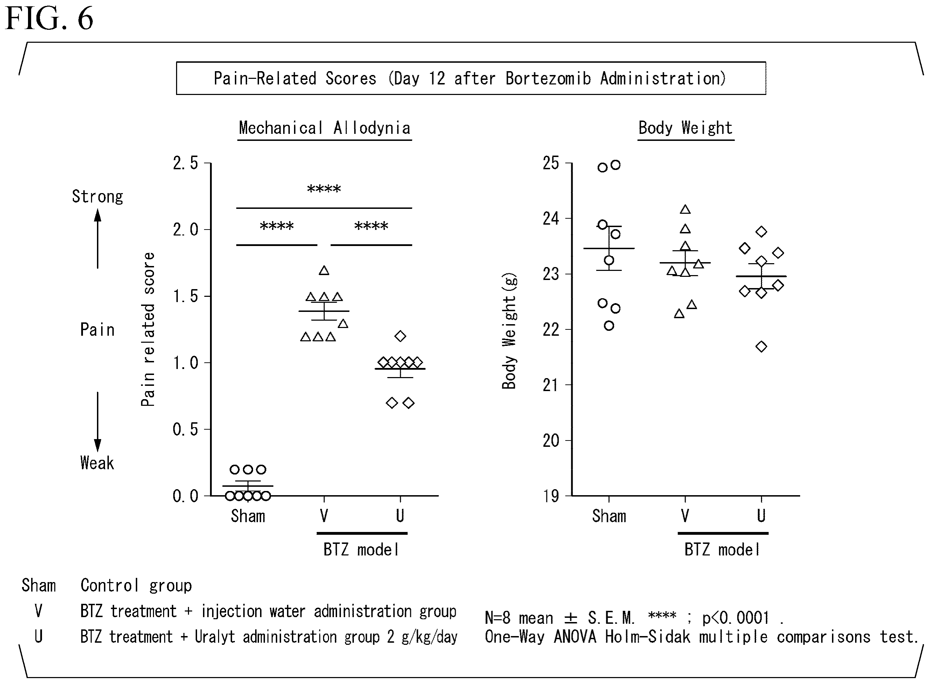

FIG. 6 is a diagram illustrating the effect of an alkalinizing agent in a bortezomib-induced mouse neuropathic pain model, and shows the pain-related score for each test group in relation to mechanical allodynia on day 12 from the start of bortezomib administration, and the body weight of each test group on day 12 from the start of bortezomib administration.

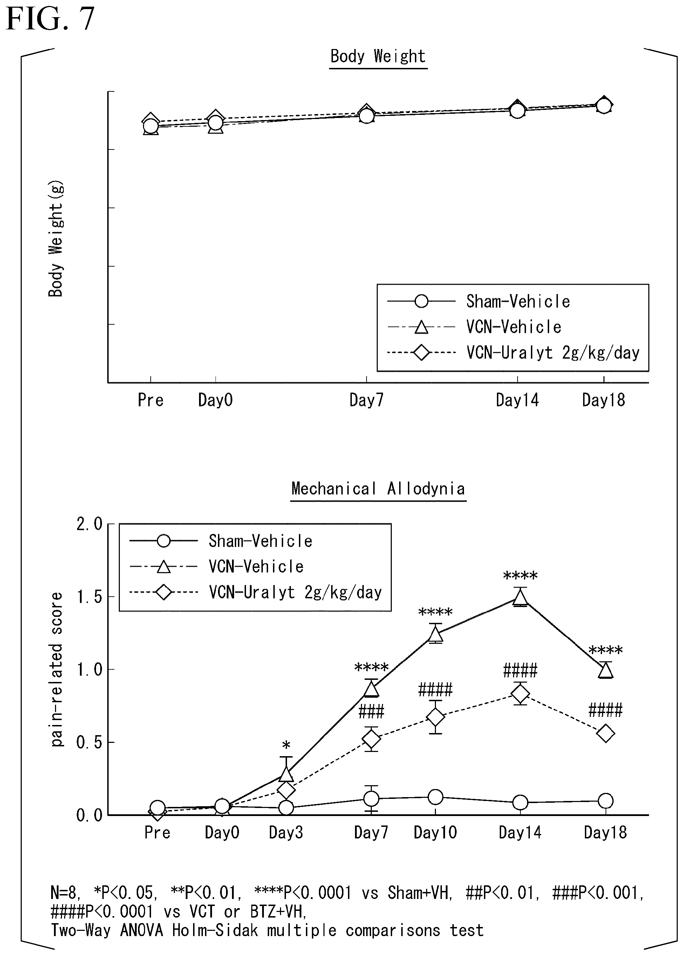

FIG. 7 is a diagram illustrating the effect of an alkalinizing agent in a vincristine-induced mouse neuropathic pain model, and shows the relationship between the number of days elapsed from the start of vincristine administration and the body weight, and the relationship between the number of days elapsed from the start of vincristine administration and the pain-related score in relation to mechanical allodynia.

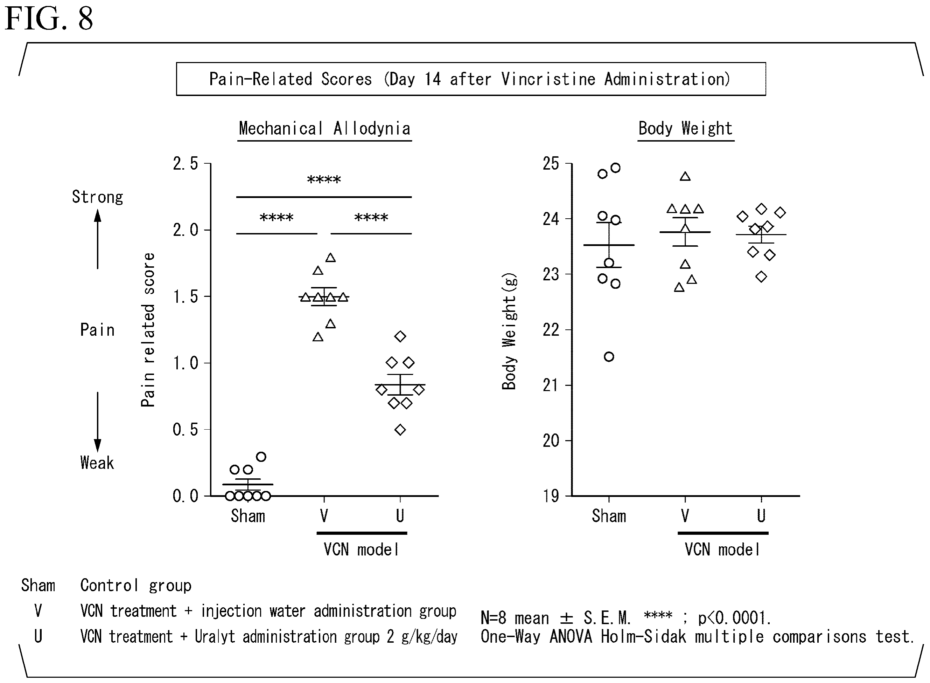

FIG. 8 is a diagram illustrating the effect of an alkalinizing agent in a vincristine-induced mouse neuropathic pain model, and shows the pain-related score for each test group in relation to mechanical allodynia on day 14 from the start of vincristine administration, and the body weight of each test group on day 14 from the start of vincristine administration.

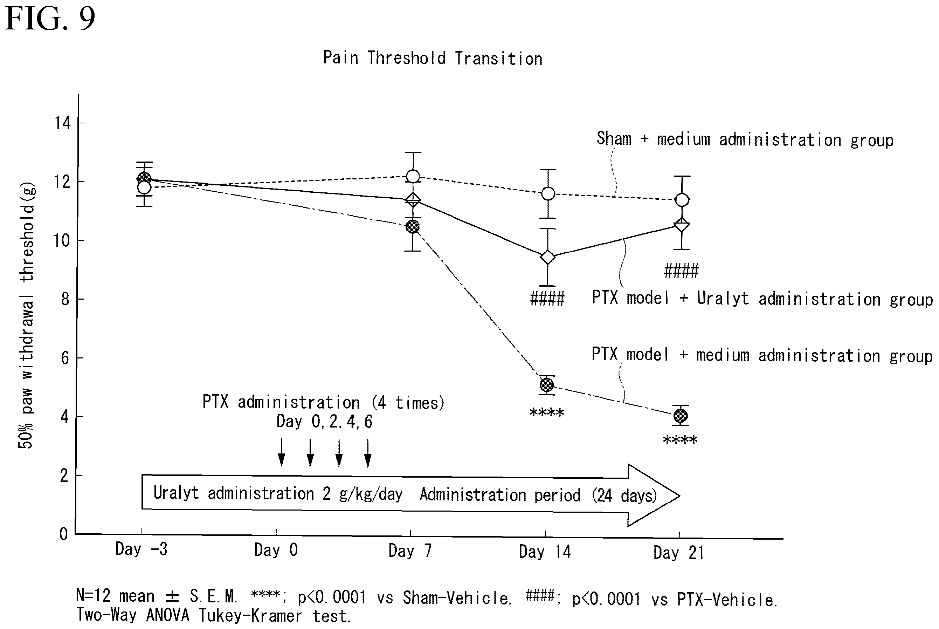

FIG. 9 is a diagram illustrating the effect of an alkalinizing agent in a paclitaxel-induced rat neuropathic pain model, and shows the relationship between the number of days elapsed from the start of paclitaxel administration and the 50% paw withdrawal threshold.

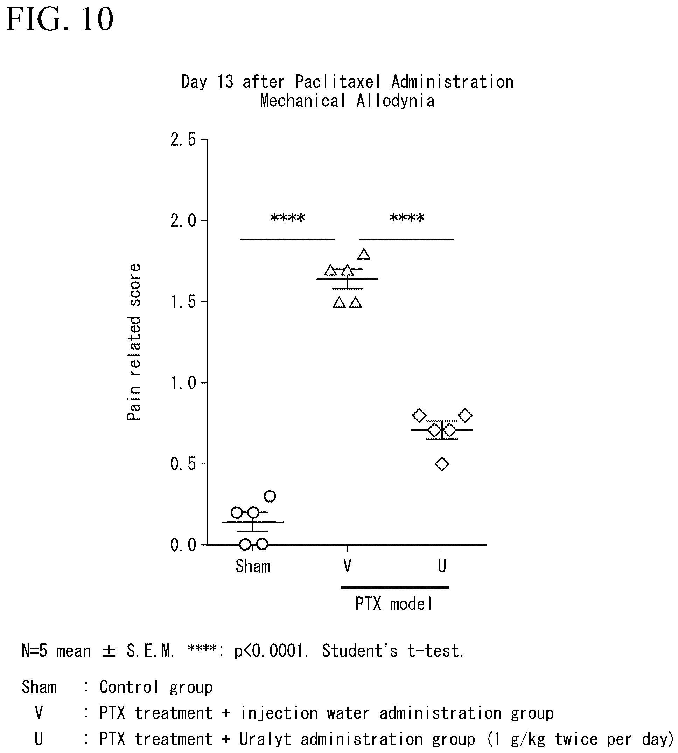

FIG. 10 is a diagram illustrating the effect of an alkalinizing agent in a paclitaxel-induced mouse neuropathic pain model, and shows the pain-related score for each test group in relation to mechanical allodynia on day 13 from the start of paclitaxel administration.

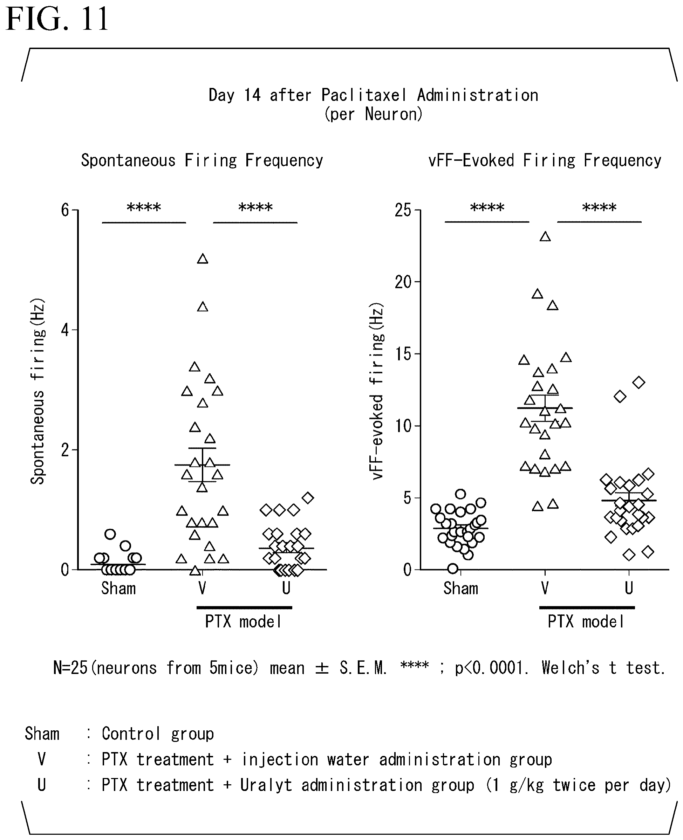

FIG. 11 is a diagram illustrating the effect of an alkalinizing agent in a paclitaxel-induced mouse neuropathic pain model, and shows the spontaneous firing frequency per neuron in each test group on day 14 from the start of paclitaxel administration, and the vFF-evoked firing frequency per neuron in each test group on day 14 from the start of paclitaxel administration.

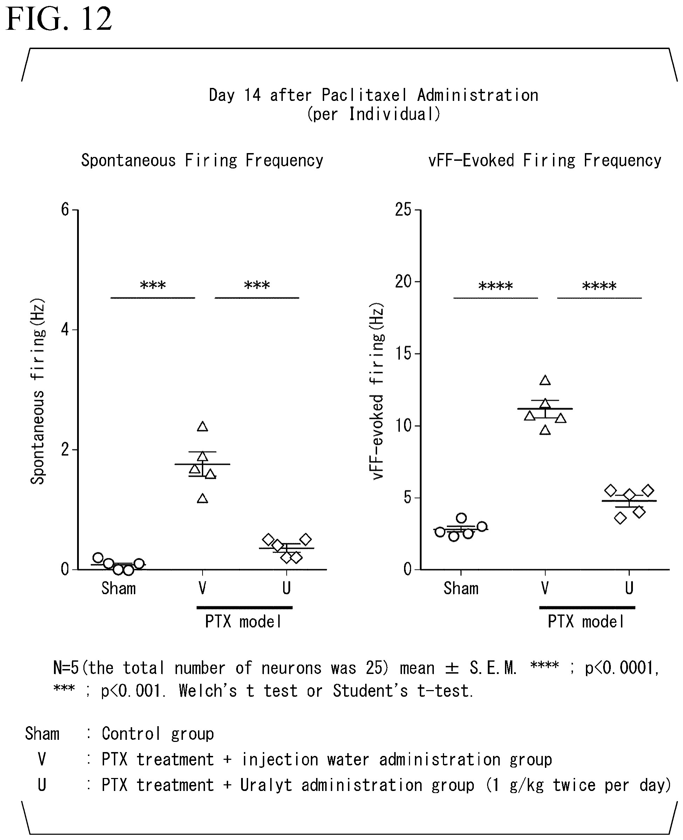

FIG. 12 is a diagram illustrating the effect of an alkalinizing agent in a paclitaxel-induced mouse neuropathic pain model, and shows the spontaneous firing frequency for each individual in each test group on day 14 from the start of paclitaxel administration, and the vFF-evoked firing frequency in each individual in each test group on day 14 from the start of paclitaxel administration.

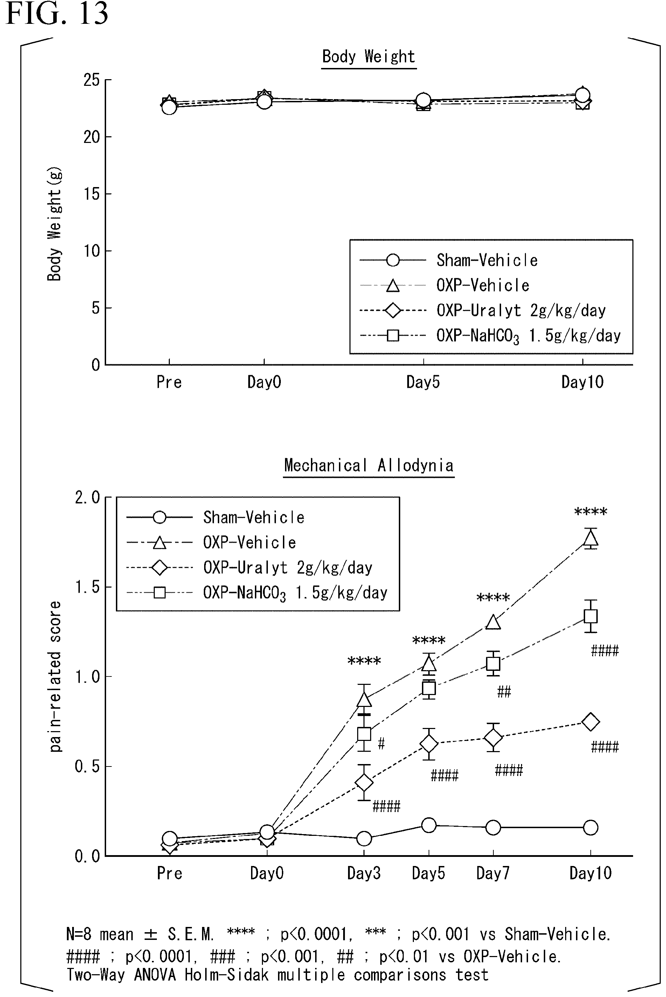

FIG. 13 is a diagram illustrating the effect of an alkalinizing agent in an oxaliplatin-induced mouse neuropathic pain model, and shows the relationship between the number of days elapsed from the start of oxaliplatin administration and the body weight, and the relationship between the number of days elapsed from the start of oxaliplatin administration and the pain-related score in relation to mechanical allodynia.

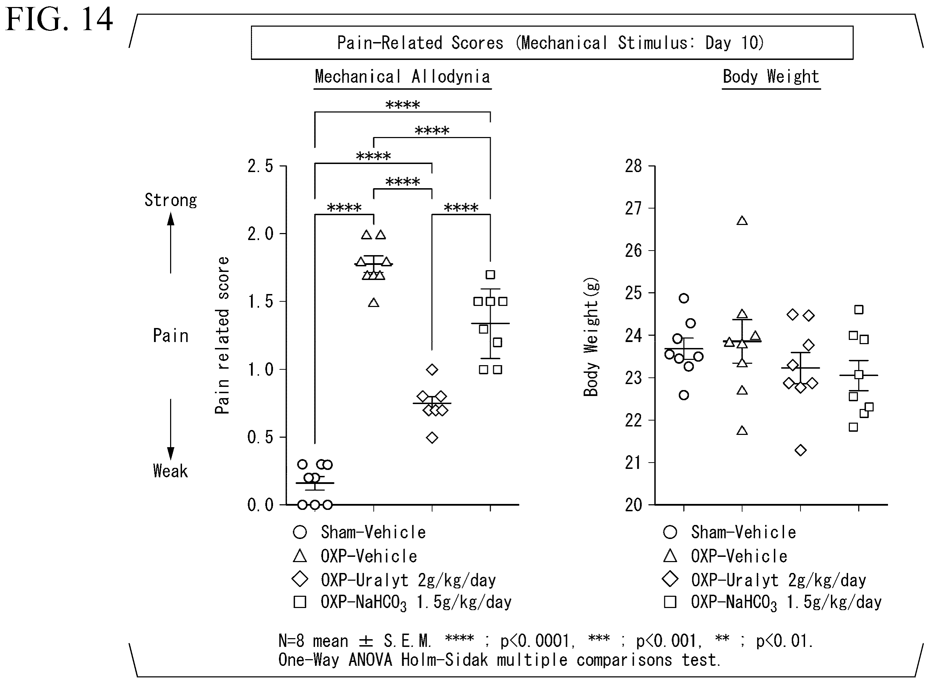

FIG. 14 is a diagram illustrating the effect of an alkalinizing agent in an oxaliplatin-induced mouse neuropathic pain model, and shows the pain-related score for each test group in relation to mechanical allodynia on day 10 from the start of oxaliplatin administration, and the body weight of each test group on day 10 from the start of oxaliplatin administration.

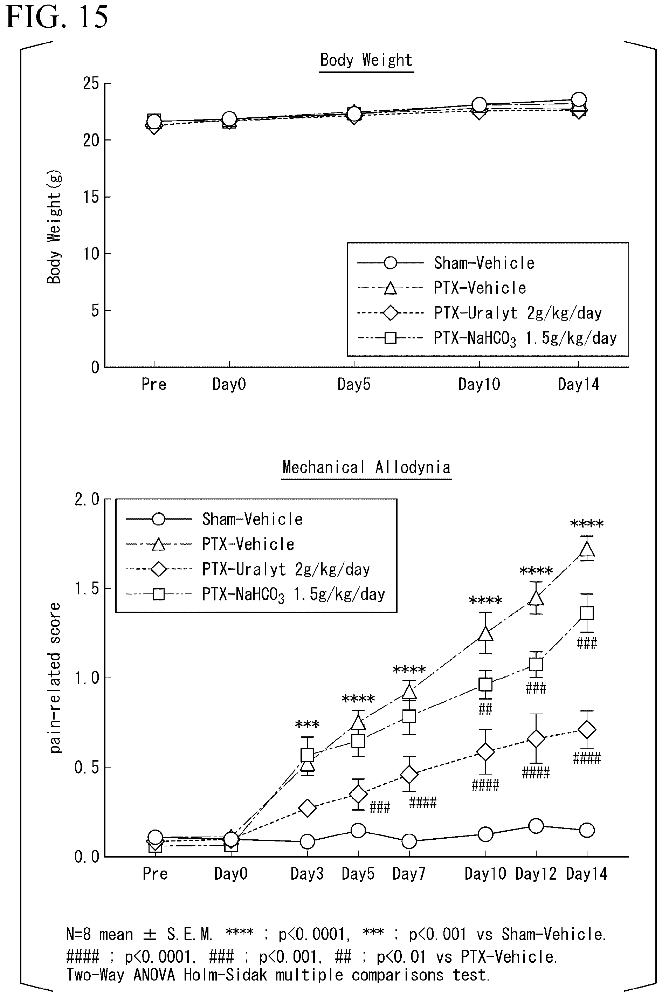

FIG. 15 is a diagram illustrating the effect of an alkalinizing agent in a paclitaxel-induced mouse neuropathic pain model, and shows the relationship between the number of days elapsed from the start of paclitaxel administration and the body weight, and the relationship between the number of days elapsed from the start of paclitaxel administration and the pain-related score in relation to mechanical allodynia.

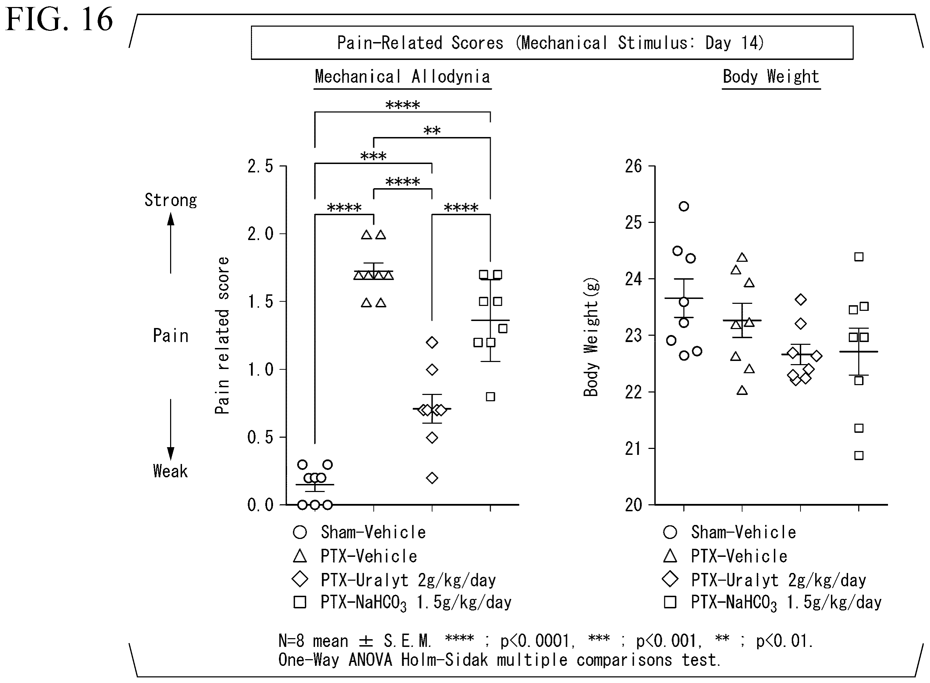

FIG. 16 is a diagram illustrating the effect of an alkalinizing agent in a paclitaxel-induced mouse neuropathic pain model, and shows the pain-related score for each test group in relation to mechanical allodynia on day 14 from the start of paclitaxel administration, and the body weight of each test group on day 14 from the start of paclitaxel administration.

### [Description of Embodiments]

### 1. Pharmaceutical Composition

### <Pharmaceutical Composition for Treating or Preventing Peripheral Neuropathy Induced by Chemotherapy Drug>

A pharmaceutical composition, provided in the present invention, for treating or preventing peripheral neuropathy induced by a chemotherapy drug may contain an alkalinizing agent as an active ingredient.

The expression "contain an alkalinizing agent as an active ingredient" means that in the pharmaceutical composition, the alkalinizing agent is either a main component, or functions as a substantial active component in the treatment or prevention of peripheral neuropathy induced by a chemotherapy drug, and is included in an amount that is effective for the above treatment or prevention.

Alkalinizing agents are drugs that have the ability to increase the HCO₃⁻ concentration or pH in the bodily fluids such as the blood or urine of mammals (and particularly humans). Examples of alkalinizing agents include pharmaceutically acceptable salts of citric acid or hydrates of those salts, or mixtures thereof, and sodium bicarbonate (baking soda). Examples of the pharmaceutically acceptable salts of citric acid include alkali metal citrate salts. Specific examples of these alkali metal citrate salts include potassium citrate and sodium citrate, which may exist as stable hydrates such as potassium citrate monohydrate (C₆H₅K₃O₇·H₂O) and sodium citrate dihydrate (C₆H₅Na₃O₇·2H₂O) respectively.

Examples of preferred alkalinizing agents for inclusion in the pharmaceutical composition provided by the present invention include sodium citrate, potassium citrate, the hydrates of these salts, or mixtures thereof, and for example, a mixture of potassium citrate monohydrate (C₆H₅K₃O₇·H₂O) and sodium citrate dihydrate (C₆H₅Na₃O₇·2H₂O) may be used. The mixing ratio between the potassium citrate monohydrate (C₆H₅K₃O₇·H₂O) and sodium citrate dihydrate (C₆H₅Na₃O₇·2H₂O) may be determined appropriately by a person skilled in the art, and for example, the molar ratio between the potassium citrate monohydrate and the sodium citrate dihydrate may be set such that for each 1 mol of potassium citrate monohydrate, the amount of sodium citrate dihydrate is within a range from 0.01 to 100 mol. The molar ratio between the potassium citrate (for example, potassium citrate monohydrate) and the sodium citrate (for example, sodium citrate dihydrate) may be set appropriately by a person skilled in the art, and may be within a range from 0.85:1.15 to 1.15:0.85, from 0.90:1.10 to 1.10:0.90, from 0.95:1.05 to 1.05:0.95, or from 0.99:1.01 to 1.01:0.99, and is preferably 1:1.

Further, another example of the active ingredient included in the pharmaceutical composition provided by the present invention is sodium citrate or the hydrate thereof, and for example, sodium citrate dihydrate (C₆H₅Na₃O₇·2H₂O) may be used.

Furthermore, yet another example of the active ingredient included in the pharmaceutical composition provided by the present invention is potassium citrate or the hydrate thereof, and for example, potassium citrate monohydrate (C₆H₅K₃O₇·H₂O) may be used.

In one embodiment, the alkalinizing agent included in the pharmaceutical composition of the present invention may contain a mixture of sodium citrate or a hydrate thereof, and potassium citrate or a hydrate thereof.

In one embodiment, the alkalinizing agent included in the pharmaceutical composition of the present invention may contain a mixture of sodium citrate or a hydrate thereof and citric acid (for example, anhydrous citric acid), or a mixture of potassium citrate or a hydrate thereof and citric acid (for example, anhydrous citric acid).

In one embodiment, the alkalinizing agent included in the pharmaceutical composition of the present invention may be a mixture of potassium citrate or a hydrate thereof, sodium citrate or a hydrate thereof, and citric acid (for example, anhydrous citric acid). In such a case, the mixing ratio (molar ratio) between the citric acid (for example, anhydrous citric acid), the potassium citrate and the sodium citrate may be determined appropriately by a person skilled in the art, and for example, may be within a range from 1:1.7 to 2.3:1.7 to 2.3, from 1:1.9 to 2.1:1.9 to 2.1, or from 1:1.95 to 2.05:1.95 to 2.05, and is preferably 1:2:2.

In one embodiment, the alkalinizing agent included in the pharmaceutical composition of the present invention may be a mixture of potassium citrate monohydrate (C₆H₅K₃O₇·H₂O), sodium citrate dihydrate (C₆H₅Na₃O₇·2H₂O), and anhydrous citric acid. In such a case, the mixing ratio (molar ratio) between the anhydrous citric acid, the potassium citrate monohydrate (C₆H₅K₃O₇·H₂O) and the sodium citrate dihydrate (C₆H₅Na₃O₇·2H₂O) may be determined appropriately by a person skilled in the art, and for example, may be within a range from 1:1.7 to 2.3:1.7 to 2.3, from 1:1.9 to 2.1:1.9 to 2.1, or from 1:1.95 to 2.05:1.95 to 2.05, and is preferably 1:2:2.

In one embodiment, the alkalinizing agent included in the pharmaceutical composition of the present invention may be composed solely of a mixture of sodium citrate or a hydrate thereof and potassium citrate or a hydrate thereof.

In this description, when weights are mentioned for citric acid, a pharmaceutically acceptable salt of citric acid or a hydrate thereof, or a mixture thereof (for example, potassium citrate monohydrate (C₆H₅K₃O₇·H₂O) and sodium citrate dihydrate (C₆H₅Na₃O₇·2H₂O)), those weights may be dry weights.

In one embodiment, treatment or prevention of peripheral neuropathy induced by a chemotherapy drug may refer to the treatment or prevention of an organic disorder of the peripheral nerves induced by the chemotherapy drug.

In another embodiment, treatment or prevention of peripheral neuropathy induced by a chemotherapy drug may refer to the treatment or prevention of symptoms attributable to peripheral neuropathy induced by the chemotherapy drug, such as pain, sensory disturbance, mobility impairment, autonomic neuropathy, or a complication thereof.

In one embodiment, the above expression "peripheral neuropathy induced by a chemotherapy drug" may refer to pain, sensory disturbance, mobility impairment, autonomic neuropathy, or a complication thereof attributable to peripheral neuropathy induced by the chemotherapy drug.

In another embodiment, the expression "peripheral neuropathy induced by a chemotherapy drug" may refer to peripheral neuropathic pain induced by the chemotherapy drug.

Further, in yet another embodiment, the above term "peripheral neuropathic pain" may refer to shooting pain, burning pain, pain (excluding shooting pain and burning pain), or tingling.

In one embodiment, the above expression "peripheral neuropathy induced by a chemotherapy drug" may refer to a sensory disturbance such as hypoesthesia, hyperesthesia or a sensory disorder that is attributable to peripheral neuropathy induced by the chemotherapy drug. Hyperesthesia is a general concept that includes allodynia, and is a concept that includes mechanical allodynia caused by mechanical stimulus. Further, the above term "sensory disorder" is a general concept that includes dysesthesia and paresthesia, and is a concept that includes cold dysesthesia caused by cold stimulus. Further, one aspect of sensory disturbance and/or mobility impairment is numbness. Accordingly, the pharmaceutical composition provided by the present invention can be used for treating or preventing numbness attributable to peripheral neuropathy induced by a chemotherapy drug.

In one embodiment, the above expression "peripheral neuropathy induced by a chemotherapy drug" may refer to autonomic neuropathy attributable to peripheral neuropathy induced by the chemotherapy drug. Typical symptoms caused by autonomic neuropathy include urinary disturbance, sweating disorder, orthostatic hypotension, constipation, or paralytic ileus.

In one embodiment, the above expression "peripheral neuropathy induced by a chemotherapy drug" refers to the manifestation of symptoms in the four limbs of a mammal (and particularly a human), and in particular the manifestation of symptoms at the limb extremities.

In one embodiment, administration of the pharmaceutical composition provided by the present invention and containing an alkalinizing agent as an active ingredient can enable treatment or prevention of peripheral neuropathy induced by a chemotherapy drug.

In one embodiment, administration of the pharmaceutical composition provided by the present invention and containing an alkalinizing agent as an active ingredient can enable treatment or prevention of pain, sensory disturbance, mobility impairment, autonomic neuropathy, or a complication thereof attributable to peripheral neuropathy induced by a chemotherapy drug.

In this description, pain attributable to peripheral neuropathy is sometimes referred to as peripheral neuropathic pain.

There are no particular limitations on the types or degree of peripheral neuropathic pain induced by a chemotherapy drug that can be treated or prevented by the pharmaceutical composition provided by the present invention and containing an alkalinizing agent as an active ingredient, but typically, pain described as shooting pain, burning pain, pain (excluding shooting pain and burning pain), or tingling can be treated or prevented.

In this description, sensory disturbance attributable to peripheral neuropathy induced by a chemotherapy drug is a general concept that includes hypoesthesia, hyperesthesia and sensory disorders. Further, hyperesthesia includes allodynia, and sensory disorder is a concept that includes dysesthesia and paresthesia. Accordingly, administration of the pharmaceutical composition provided by the present invention and containing an alkalinizing agent as an active ingredient can enable treatment or prevention of sensory disturbance such as hypoesthesia, hyperesthesia and sensory disorders, including allodynia, dysesthesia and paresthesia, attributable to peripheral neuropathy induced by a chemotherapy drug.

In one embodiment, administration of the pharmaceutical composition provided by the present invention and containing an alkalinizing agent as an active ingredient can enable treatment or prevention of symptoms attributable to autonomic neuropathy induced by a chemotherapy drug, such as urinary disturbance, sweating disorder, orthostatic hypotension, constipation, or paralytic ileus.

In one embodiment, administration of the pharmaceutical composition provided by the present invention and containing an alkalinizing agent as an active ingredient can enable treatment or prevention of symptoms that manifest in the four limbs of a mammal (and particularly a human), and particularly symptoms that manifest at the limb extremities.

In this description, there are no particular limitations on the "chemotherapy drug", provided it can induce peripheral neuropathy, and examples include "cancer chemotherapy drugs". Further, there are no particular limitations on this "cancer chemotherapy drug", provided it can induce peripheral neuropathy.

In the technical field, it is known that the cancer chemotherapy drugs listed below have a tendency to induce peripheral neuropathy.

Cytotoxic anticancer drugs such as paclitaxel, docetaxel, cabazitaxel, vinorelbine, vincristine, vinblastine, vindesine, eribulin, oxaliplatin, carboplatin, cisplatin and nelarabine.

Molecular targeted anticancer drugs such as bortezomib, ixazomib, romidepsin, gilteritinib, trastuzumab emtansine, brentuximab vedotin, obinutuzumab, blinatumomab, lorlatinib, entrectinib, encorafenib, binimetinib and pemigatinib.

Immunotherapy drugs such as nivolumab, ipilimumab, pembrolizumab, atezolizumab and avelumab.

Other anticancer drugs such as cytarabine, ifosfamide, nedaplatin, thalidomide, lenalidomide and pomalidomide.

Here, the terms "cytotoxic anticancer drugs", "molecular targeted anticancer drugs", "immunotherapy drugs" and "other anticancer drugs" are used merely to classify the chemotherapy drugs listed above, but use of those terms in no way limits the types of drugs belonging to each class.

Accordingly, in this description, "peripheral neuropathy induced by a chemotherapy drug" may refer to peripheral neuropathy induced by at least one anticancer drug selected from the group consisting of cytotoxic anticancer drugs such as paclitaxel, docetaxel, cabazitaxel, vinorelbine, vincristine, vinblastine, vindesine, eribulin, oxaliplatin, carboplatin, cisplatin and nelarabine; molecular targeted anticancer drugs such as bortezomib, ixazomib, romidepsin, gilteritinib, trastuzumab emtansine, brentuximab vedotin, obinutuzumab, blinatumomab, lorlatinib, entrectinib, encorafenib, binimetinib and pemigatinib; immunotherapy drugs such as nivolumab, ipilimumab, pembrolizumab, atezolizumab and avelumab; and other anticancer drugs such as cytarabine, ifosfamide, nedaplatin, thalidomide, lenalidomide and pomalidomide.

In one embodiment, administration of the pharmaceutical composition provided by the present invention and containing an alkalinizing agent as an active ingredient can enable treatment or prevention of peripheral neuropathy induced by a cancer chemotherapy drug listed above, namely an anticancer drug.

In another embodiment, administration of the pharmaceutical composition provided by the present invention and containing an alkalinizing agent as an active ingredient can enable treatment or prevention of peripheral neuropathy induced by at least one anticancer drug selected from the group consisting of paclitaxel, vincristine, oxaliplatin and bortezomib.

In yet another embodiment, administration of the pharmaceutical composition provided by the present invention and containing an alkalinizing agent as an active ingredient can enable treatment or prevention of peripheral neuropathy induced by paclitaxel or oxaliplatin.

In yet another embodiment, administration of the pharmaceutical composition provided by the present invention and containing an alkalinizing agent as an active ingredient can enable treatment or prevention of peripheral neuropathy induced by bortezomib or vincristine.

In this description, the term "treatment" is a concept that incorporates the elimination of, complete recovery from, curing of, or remission from a "pathological" or "abnormal" symptom, state or disease, and actions or measures therefor, incorporates the "suppression" of any worsening of a "pathological" or "abnormal" symptom, state or disease, and actions or measures therefor, and also incorporates the concept of "improvement".

In one embodiment, the term "treatment" refers to the elimination of, complete recovery from, curing of, or remission from a "pathological" or "abnormal" symptom, state or disease, or an action or measure for achieving that outcome. In another embodiment, the term "treatment" refers to the elimination of, complete recovery from, curing of, or remission from a "pathological" or "abnormal" symptom, state or disease.

In one embodiment, "treatment" includes the administration of the pharmaceutical composition provided by the present invention and containing an alkalinizing agent as an active ingredient as one part of a treatment with a cancer chemotherapy drug described above, namely an anticancer drug.

In one embodiment, "treatment" may include the concept of starting administration of the pharmaceutical composition provided by the present invention and containing an alkalinizing agent as an active ingredient prior to the administration of a cancer chemotherapy drug described above, namely an anticancer drug, thereby enabling the elimination of, complete recovery from, curing of, or remission from a "pathological" or "abnormal" symptom, state or disease caused by the administration of the cancer chemotherapy drug, namely the anticancer drug, and actions or measures therefor, or enabling the "suppression" of any worsening of a "pathological" or "abnormal" symptom, state or disease caused by the administration of the cancer chemotherapy drug, namely the anticancer drug, and actions or measures therefor, or enabling "improvement".

In one embodiment, in the case where administration of the pharmaceutical composition provided by the present invention and containing an alkalinizing agent as an active ingredient is started prior to the administration of a cancer chemotherapy drug described above, namely an anticancer drug, the administration may be started within a range from 10 days to one day prior, 7 days to one day prior, or 3 days to one day prior, to the administration of the cancer chemotherapy drug, namely the anticancer drug.

In this description, the term "suppression" is a concept that incorporates halting, reducing the speed of, or decreasing the worsening or progression of a symptom, state or disease, and actions or measures therefor, or incorporates improvement in a symptom, state or disease, and actions or measures therefor. Here, the term "improvement" includes the change in a "pathological" or "abnormal" symptom, state or disease toward a more "healthy" or "normal" state, and actions or measures therefor, or achieving a "healthy" or "normal state", and actions or measures therefor. Accordingly, in one embodiment, the term "improvement" includes a change in a numerical value that acts as an indicator of a "pathological" or "abnormal" symptom or state, with the numerical value becoming smaller or larger, and either approaching a normal value or reaching a normal value, as a result of the "improvement". The above expression "worsening or progression of a symptom, state or disease" includes the worsening or progression of a "pathological" or "abnormal" symptom, state or disease, and worsening or progression from a "healthy" or "normal" state to a "pathological" or "abnormal" symptom, state or disease. In one embodiment, the term "suppression" refers to halting, reducing the speed of, or decreasing the worsening or progression of a symptom, state or disease, or an action or measure therefor. In another embodiment, the term "suppression" refers to halting, reducing the speed of, or decreasing the worsening or progression of a symptom, state or disease.

In this description, the term "healthy" describes a state of having no acute or chronic diseases or disorders, and the term "normal" describes a state typically expressed by a healthy subject.

In this description, the term "prevention" is a concept that incorporates preventing onset of a "pathological" or "abnormal" symptom, state or disease before it occurs, and actions or measures therefor. Accordingly, in a state with no onset (also referred to as expression) of a "pathological" or "abnormal" symptom, state or disease, "suppression" of this symptom, state or disease may include the concept of "prevention".

The symptom, state or disease mentioned above is either compared before administration and then after administration of the pharmaceutical composition provided by the present invention, or compared for the case when the pharmaceutical composition provided by the present invention is administered and the case when a control or a placebo is administered.

Accordingly, for example, the expression "treatment of peripheral neuropathic pain induced by a chemotherapy drug" is a concept that includes the elimination of, complete recovery from, curing of, or remission from the peripheral neuropathic pain induced by the chemotherapy drug, and actions or measures therefor, includes suppression of the peripheral neuropathic pain induced by the chemotherapy drug, and actions or measures therefor, or includes improvement in the peripheral neuropathic pain induced by the chemotherapy drug. In another aspect, the above treatment is a concept that incorporates suppression of a symptom or state of the above peripheral neuropathic pain induced by the chemotherapy drug following administration of the pharmaceutical composition provided by the present invention and containing an alkalinizing agent as an active ingredient, compared with the symptom or state of the pain prior to administration, or incorporates suppression of the symptom or state of the above peripheral neuropathic pain by administration of the pharmaceutical composition provided by the present invention and containing an alkalinizing agent as an active ingredient, compared with a placebo administration or a control.

Furthermore, for example, the expression "prevention of peripheral neuropathic pain induced by a chemotherapy drug" is a concept that incorporates preventing onset of peripheral neuropathic pain induced by the chemotherapy drug before it occurs, and actions or measures therefor, and also incorporates suppressing the pain prior to pain onset, and actions or measures therefor. In another aspect, when the pharmaceutical composition provided by the present invention and containing an alkalinizing agent as an active ingredient is administered prior to the onset of peripheral neuropathic pain induced by a chemotherapy drug, even if the pain does occur, provided a symptom or state of that pain is suppressed compared with a placebo administration or a control, that phenomenon may be included within the definition of prevention.

In the case of "pain", the effect of the above "suppression" can be evaluated based on the "pain-related score" described below compared with a placebo administration or control.

By substituting the term "pain" above with a symptom or state of peripheral neuropathy induced by a chemotherapy drug described above, the concepts of treatment and prevention can be understood. In terms of evaluation of effects, other appropriate indicators may be applied in place of the above "pain-related score".

In this description, an expression "A, B and/or C" means "at least one item selected from the group consisting of A, B and C".

The pharmaceutical composition provided by the present invention and containing an alkalinizing agent as an active ingredient is administered either orally or parenterally to a human or other mammal, wherein examples of parenteral administration include intravenous administration, subcutaneous administration, intramuscular administration, intra-articular administration, transmucosal administration, transdermal administration, transnasal administration, rectal administration, intrathecal administration, intraperitoneal administration, and local administration.

The pharmaceutical composition provided by the present invention and containing an alkalinizing agent as an active ingredient may be prepared using citric acid, a pharmaceutically acceptable salt of citric acid or a hydrate thereof, or a mixture thereof; or sodium bicarbonate, either without any other components, or by mixing with a pharmaceutically acceptable carrier such as an excipient (for example, lactose, D-mannitol, crystalline cellulose, or glucose), a binder (for example, hydroxypropyl cellulose (HPC), gelatin, or polyvinylpyrrolidone (PVP)), a lubricant (for example, magnesium stearate or talc), a disintegrant (for example, starch or carboxymethyl cellulose calcium (CMC-Ca)), or a diluent (for example, injection water or physiological saline solution), and any of various additives where necessary (for example, a pH regulator, surfactant, solubilizer, preservative, emulsifier, isotonizing agent, or stabilizer), and may have any of various forms, such as tablets, capsules, suspensions, injections or suppositories. For example, a tablet may be prepared by mixing citric acid, a pharmaceutically acceptable salt of citric acid or a hydrate thereof, or a mixture thereof, or sodium bicarbonate with an excipient (for example, lactose, D-mannitol, crystalline cellulose, or glucose), a disintegrant (for example, starch or carboxymethyl cellulose calcium (CMC-Ca)), a binder (for example, hydroxypropyl cellulose (HPC), gelatin, or polyvinylpyrrolidone (PVP)) and a lubricant (for example, magnesium stearate or talc) and the like, and then tableting the mixture.

A tablet according to the present invention is described below in further detail.

In one embodiment, the pharmaceutical composition provided by the present invention and containing an alkalinizing agent as an active ingredient is a tablet. The tablet provided by the present invention may contain an active ingredient (for example, potassium citrate or a hydrate thereof, sodium citrate or a hydrate thereof, a mixture of potassium citrate monohydrate and sodium citrate dihydrate, or sodium bicarbonate), as well as any typical pharmaceutically acceptable additives used in the field of drug production. Examples of these additives include excipients, binders, disintegrants, fluidizing agents, flavorings, lubricants, pH regulators, surfactants, stabilizers, and fragrances.

The amount of the active ingredient in the tablet provided by the present invention may typically be within a range from 10 to 95% by weight relative to the tablet, and is preferably within a range from 30 to 90% by weight, and more preferably from 60 to 85% by weight.

Examples of excipients that may be used in the tablet provided by the present invention include sugars such as lactose (for example, lactose hydrate and anhydrous lactose), glucose, sucrose, fructose and maltose, sugar alcohols such as erythritol, sorbitol, maltitol, xylitol and D-mannitol, as well as starches (for example, corn starch, potato starch, rice starch and wheat starch), crystalline cellulose, magnesium aluminometasilicate, anhydrous calcium phosphate, precipitated calcium carbonate, calcium silicate, calcium lactate and ethyl cellulose, and crystalline cellulose is particularly preferred.

The amount of the excipient in the tablet provided by the present invention may typically be within a range from 1 to 95% by weight relative to the tablet, and is preferably within a range from 1 to 80% by weight, more preferably from 3 to 80% by weight, and even more preferably from 3 to 20% by weight.

Examples of binders that may be used in the tablet provided by the present invention include hydroxypropyl cellulose, hydroxypropyl methylcellulose, polyvinylpyrrolidone, dextrin, methyl cellulose, polyvinyl alcohol, sodium alginate, aminoalkyl methacrylate copolymer, polyethylene glycol, pregelatinized starch (such as partially pregelatinized starch), agar and gelatin, and hydroxypropyl cellulose is particularly preferred.

The amount of the binder in the tablet provided by the present invention may typically be within a range from 0.1 to 30% by weight relative to the tablet, and is preferably within a range from 0.1 to 10% by weight, and more preferably from 0.3 to 3% by weight.

Examples of disintegrants that may be used in the tablet provided by the present invention include croscarmellose sodium, carmellose calcium, carboxymethyl starch sodium, low-substituted hydroxypropyl cellulose, crospovidone, starch (such as wheat starch, corn starch and partially pregelatinized starch) and carmellose, and partially pregelatinized starch is particularly preferred.

The amount of the disintegrant in the tablet provided by the present invention may typically be within a range from 0.3 to 20% by weight relative to the tablet, and is preferably within a range from 1 to 10% by weight, and more preferably from 3 to 10% by weight.

Examples of fluidizing agents that may be used in the tablet provided by the present invention include light anhydrous silicic acid, talc, and magnesium aluminometasilicate.

The amount of the fluidizing agent in the tablet provided by the present invention may typically be within a range from 0.03 to 3% by weight relative to the tablet, and is preferably within a range from 0.1 to 3% by weight, and more preferably from 0.3 to 3% by weight.

Examples of flavorings that may be used in the tablet provided by the present invention include acidifiers such as citric acid (for example, anhydrous citric acid), malic acid, acetic acid, tartaric acid, fumaric acid and ascorbic acid (however, these flavorings are not included in the active ingredient of the present invention), and sweeteners such as saccharin sodium, dipotassium glycyrrhizate, aspartame (a registered trademark), stevia, thaumatin and sucralose.

The amount of the flavoring in the tablet provided by the present invention may typically be within a range from 0.03 to 3% by weight relative to the tablet, and is preferably within a range from 0.1 to 3% by weight, and more preferably from 0.3 to 3% by weight.

Examples of lubricants that may be used in the tablet provided by the present invention include magnesium stearate, calcium stearate, talc, light anhydrous silicic acid, sucrose fatty acid esters, carnauba wax, macrogol and sodium stearyl fumarate, and magnesium stearate is particularly preferred.

The amount of the lubricant in the tablet provided by the present invention may typically be within a range from 0.1 to 30% by weight relative to the tablet, and is preferably within a range from 0.3 to 10% by weight, and more preferably from 1 to 3% by weight.

Examples of pH regulators that may be used in the tablet provided by the present invention include citric acid, phosphate salts (such as sodium dihydrogen phosphate and potassium dihydrogen phosphate), carbonate salts (such as magnesium carbonate and sodium carbonate), tartrate salts, fumarate salts, acetate salts, and amino acid salts (although the pH regulator is not included in the active ingredient of the present invention).

The amount of the pH regulator in the tablet provided by the present invention may typically be within a range from 0.1 to 30% by weight relative to the tablet, and is preferably within a range from 0.3 to 10% by weight, and more preferably from 1 to 5% by weight.

Examples of surfactants that may be used in the tablet provided by the present invention include sodium lauryl sulfate, polysorbate, sucrose fatty acid esters, polyoxyethylene hydrogenated castor oil, polyoxyl stearate, macrogol, and poloxamer.

The amount of the surfactant in the tablet provided by the present invention may typically be within a range from 0.01 to 3% by weight relative to the tablet, and is preferably within a range from 0.03 to 1% by weight, and more preferably from 0.03 to 0.5% by weight.

Examples of stabilizers that may be used in the tablet provided by the present invention include citric acid (for example, anhydrous citric acid), malic acid, acetic acid, tartaric acid, maleic acid, ascorbic acid, sodium edetate and tocopherol (although the stabilizer is not included in the active ingredient of the present invention), and anhydrous citric acid is particularly preferred.

The amount of the stabilizer in the tablet provided by the present invention may typically be within a range from 0.01 to 30% by weight relative to the tablet, and is preferably within a range from 0.1 to 30% by weight, and more preferably from 1 to 20% by weight.

Examples of fragrances that may be used in the tablet provided by the present invention include citrus-based fragrances such as lemon, orange and grapefruit, as well as peppermint, spearmint and menthol, and these fragrances may be included in appropriate amounts in the tablet (for example, within a range from 0.01 to 1% by weight relative to the tablet, and preferably within a range from 0.01 to 0.1% by weight).

The combined amount of the active ingredient and these pharmaceutically acceptable additives in the tablet provided by the present invention must not exceed 100% by weight of the tablet.

The tablet provided by the present invention contains the components described above, and may be an uncoated tablet having no coating layer, or may be a film-coated tablet having a coating layer. The amount of the coating layer may be determined appropriately by a person skilled in the art, and for example, may be within a range from 0.1 to 10% by weight relative to the uncoated tablet. The coating layer may include, in addition to the coating base, appropriate amounts of a plasticizer, colorant, and/or gloss agent or the like.

Examples of coating bases that may be used on the tablet provided by the present invention include hydroxypropyl cellulose, hydroxypropyl methylcellulose, ethyl cellulose, cellulose acetate phthalate, methacrylic acid copolymers and polyvinylpyrrolidone, and hydroxypropyl methylcellulose is particularly preferred. The amount of the coating base in the tablet provided by the present invention is typically within a range from 0.01 to 10% by weight relative to the tablet, and is preferably within a range from 0.3 to 3% by weight.

Examples of coating plasticizers that may be used in the tablet provided by the present invention include triethyl citrate, medium chain fatty acid triglycerides, triacetin, glycerol, propylene glycol and polyethylene glycol (for example, macrogol 6000), and macrogol 6000 is particularly preferred. The amount of the coating plasticizer in the tablet provided by the present invention is typically within a range from 0.01 to 1% by weight relative to the tablet, and is preferably within a range from 0.03 to 3% by weight.

Examples of coating colorants that may be used in the tablet provided by the present invention include titanium oxide, yellow iron sesquioxide, iron sesquioxide, black iron oxide, food blue No. 2, and food blue No. 2 aluminum chelate. The amount of the coating colorant in the tablet provided by the present invention is typically within a range from 0.01 to 1% by weight relative to the tablet, and is preferably within a range from 0.03 to 3% by weight.

Examples of coating gloss agents that may be used in the tablet provided by the present invention include carnauba wax. The amount of the coating gloss agent in the tablet provided by the present invention is typically within a range from 0.0001 to 0.1% by weight relative to the tablet, and is preferably within a range from 0.001 to 0.01% by weight.

The pharmaceutical composition provided by the present invention and containing an alkalinizing agent as an active ingredient can be produced using known methods from the field of drug production. For example, in the case of tablets, the production method may include a mixing step of mixing the active ingredient (for example, potassium citrate or a hydrate thereof, sodium citrate or a hydrate thereof, a mixture of potassium citrate monohydrate and sodium citrate dihydrate, or sodium bicarbonate) with the additives, a granulation step, a tableting step and/or a coating step.

The mixing step may include a step of mixing the active ingredient with additives such as an excipient, stabilizer, disintegrant and/or binder or the like. Further, prior to the tableting step, the production method may also include a step of mixing the mixture containing the active ingredient and the additives with a lubricant, flavoring and/or fragrance. Mixing may be conducted using a V-type mixer, W-type mixer, container mixer, tumbler mixer, or stirring mixer or the like.

The granulation step can be conducted using known granulation methods from the field of drug production. Examples of the granulation method include dry granulation methods, wet granulation methods and fluidized bed granulation methods.

In one embodiment, the mixture obtained in the mixing step or the granules obtained in the granulation step may be appropriately crushed and/or classified to obtain a mixture or granules having a desired particle size. Crushing can be conducted using the types of crushing devices known in the field of drug production, such as a ball mill, jet mill, or hammer mill or the like. Classification may be conducted using a sieve within a range from a 16-mesh sieve (mesh opening: 1,000 µm) to a 32-mesh sieve (mesh opening: 500 µm).

The tableting step can be conducted using tableting methods known in the field of drug production. Examples of the tableting method include direct tableting methods, dry tableting methods, wet tableting methods, and external lubricant tableting methods. For example, by using a tableting machine known in the field of drug production, such as a single-shot tableting machine or a rotary tableting machine, the mixture or granules obtained in the step described above can be tableted. In those cases where a single-shot tableting machine or rotary tableting machine or the like is used, a tableting pressure within a range from 1 kN to 30 kN may be used.

The coating step may be conducted using known methods from the field of drug production. For example, the coating step may be conducted by spray coating a coating liquid containing the coating base and appropriate amounts of a plasticizer, colorant and/or gloss agent and the like onto the exterior of the uncoated tablets.

In one embodiment, the tablet provided by the present invention can be produced by mixing the active ingredient, an excipient (for example, lactose, D-mannitol, crystalline cellulose and/or glucose), a binder (for example, hydroxypropyl cellulose (HPC), gelatin and/or polyvinylpyrrolidone (PVP)), a stabilizer (for example, anhydrous citric acid), a disintegrant (for example, starch (such as partially pregelatinized starch) and/or carboxymethyl cellulose calcium (CMC-Ca)), and a lubricant (for example, magnesium stearate), tableting the mixture to obtain uncoated tablets, and forming a coating layer containing a coating base (for example, hydroxypropyl cellulose, hydroxypropyl methylcellulose and/or PVP), a plasticizer (for example, triethyl citrate and/or macrogol 6000), a colorant (for example, iron sesquioxide and/or titanium oxide) and a gloss agent (for example, carnauba wax) on the exterior surfaces of the uncoated tablets.

In one embodiment, the hardness of the obtained tablets may be within a range from 10 to 200 N, and preferably from 30 to 150 N.

In the pharmaceutical composition provided by the present invention and containing an alkalinizing agent as an active ingredient, the amount of the alkalinizing agent may be set as appropriate.

In one embodiment, the amount of the alkalinizing agent in the pharmaceutical composition provided by the present invention and containing the alkalinizing agent as an active ingredient may be set to an amount that results in an improvement in aciduria in gout or hyperuricemia when the dosage of the alkalinizing agent is administered to a human, or may be set to a smaller amount or a larger amount. For example, the amount of the alkalinizing agent may be set to a value that is within a range from 100 to 800% by mass, from 200 to 800% by mass, or from 400 to 800% by mass, of the daily dosage recognized in Japan as providing improvement in aciduria in gout or hyperuricemia (for example, in the case where a citric acid formulation is used as the alkalinizing agent: tablets each containing 231.5 mg of potassium citrate (C₆H₅K₃O₇·H₂O) and 195.0 mg of sodium citrate hydrate (C₆H₅Na₃O₇·2H₂O) are administered orally at a rate of two tablets per dosage and three doses per day; or in the case where sodium bicarbonate is used as the alkalinizing agent: 3 to 5 g administered orally per day).

In one embodiment, the pharmaceutical composition provided by the present invention and containing an alkalinizing agent as an active ingredient may be in the form of a tablet, wherein each tablet contains potassium citrate monohydrate or sodium citrate dihydrate as the active ingredient in an amount within a range from 10 mg to 1 g, preferably from 100 mg to 500 mg, and more preferably from 400 to 500 mg.

In one embodiment, the pharmaceutical composition provided by the present invention and containing an alkalinizing agent as an active ingredient may be in the form of a tablet, wherein each tablet contains potassium citrate monohydrate and sodium citrate dihydrate in respective amounts within a range from 10 mg to 300 g and a combined amount of 20 mg to 600 mg, preferably in respective amounts from 150 mg to 250 mg and a combined amount of 400 mg to 500 mg, and more preferably in respective amounts from 190 to 240 mg and a combined amount of 400 mg to 450 mg.

In one embodiment, the pharmaceutical composition provided by the present invention and containing an alkalinizing agent as an active ingredient may be in the form of a tablet, wherein each tablet contains sodium bicarbonate as the alkalinizing agent in an amount within a range from 10 mg to 1 g, and preferably from 100 mg to 500 mg.

In one embodiment, the pharmaceutical composition provided by the present invention and containing an alkalinizing agent as an active ingredient may be in the form of a tablet, wherein each tablet contains 231.5 mg of potassium citrate monohydrate and 195.0 mg of sodium citrate dihydrate as the active ingredients, and contains anhydrous citric acid, crystalline cellulose, partially pregelatinized starch, hydroxypropyl cellulose, magnesium stearate, hypromellose, macrogol 6000, titanium oxide and carnauba wax as additives.

In one embodiment, a tablet containing 231.5 mg of potassium citrate monohydrate and 195.0 mg of sodium citrate dihydrate may be used as a single dosage unit.

In this description, a "dosage unit" indicates a unit of the formulation, wherein a "single dosage unit" represents the smallest unit of the formulation. Accordingly, in the case of tablets, the dosage unit is each tablet, and a single dosage unit indicates one tablet. In the case of an injection, the dosage unit is an injection used to fill a sealed container such as an ampule or vial, and a single dosage unit indicates the injection contained within a single sealed container such as an ampule or vial. In the case of a powder or jelly-like formulation, the dosage unit is the powder or jelly-like formulation sealed in a bag produced using a single-layer or multilayer film-like sheet of polyethylene or the like, or a similar sheet provided with an aluminum layer, and a single dosage unit indicates the powder or jelly-like formulation sealed in a single bag. In the case of capsules (hard capsules, soft capsules, and seamless capsules and the like), the dosage unit is each capsule, and a single dosage unit indicates one capsule.

In those cases where the pharmaceutical composition provided by the present invention is administered to a human or other mammal, one or two or more of the above dosage units may be administered per dose, or a single dosage unit may be divided and only a portion administered.

The dosage of the active ingredient may be set appropriately in accordance with factors such as the type of active ingredient, the administration method, the age, weight, gender, symptoms and drug sensitivity of the administration subject, the dosage schedule for the chemotherapy drug, and the degree of peripheral neuropathy induced by the chemotherapy drug, and typically the dosage may be adjusted in accordance with the dosage schedule for the chemotherapy drug, the degree of peripheral neuropathy induced by the chemotherapy drug, and the level of improvement in the symptoms.

In one embodiment, in the case where a mixture of potassium citrate monohydrate and sodium citrate dihydrate is administered orally as the active ingredient to a human, the potassium citrate monohydrate and sodium citrate dihydrate may be administered using respective dosages of 1.5 to 6 g/day and a combined amount of 3 to 12 g/day, respective dosages of 1.5 to 9 g/day and a combined amount of 3 to 18 g/day, or respective dosages of 1.5 to 12 g/day and a combined amount of 3 to 24 g/day, and preferably respective dosages of 3 to 12 g/day and a combined amount of 6 to 24 g/day, respective dosages of 3 to 9 g/day and a combined amount of 6 to 18 g/day, or respective dosages of 6 to 9 g/day and a combined amount of 12 to 18 g/day, wherein the administration may be performed between one and five times per day, and is preferably divided into three doses per day.

In one embodiment, in the case where either potassium citrate monohydrate or sodium citrate dihydrate is administered orally as the active ingredient to a human, administration may be conducted using a dosage of 3 to 24 g/day, 3 to 18 g/day, or 3 to 12 g/day, and the administration may be performed between one and five times per day, and is preferably divided into three doses per day.

In one embodiment, in the case where sodium bicarbonate is administered orally as the active ingredient to a human, administration may be conducted using a dosage of 2.25 to 9 g/day, and the administration may be performed between one and five times per day, and is preferably divided into three doses per day.

In one embodiment, the pharmaceutical composition provided by the present invention and containing an alkalinizing agent as an active ingredient may be administered prior to the administration of a chemotherapy drug to a subject scheduled for chemotherapy and at risk of developing peripheral neuropathy with the object of preventing the onset of peripheral neuropathy induced by the chemotherapy drug, or may be administered prior to administration of the chemotherapy drug, with administration continued after administration of the chemotherapy drug, or may be administered to a subject suffering from peripheral neuropathy induced by a chemotherapy drug with the object of treating the peripheral neuropathy. The administration may be continued in accordance with the dosage schedule for the chemotherapy drug, the degree of peripheral neuropathy induced by the chemotherapy drug, and the level of improvement in the symptoms.

In one embodiment, the pharmaceutical composition provided by the present invention and containing an alkalinizing agent as an active ingredient may be administered for a long period of time, and for example, may be administered for one week, 2 weeks, 3 weeks, 6 weeks, 8 weeks, 10 weeks, 12 weeks, 24 weeks, 40 weeks, 60 weeks, 80 weeks, 100 weeks, 120 weeks, at least one week, at least 2 weeks, at least 3 weeks, at least 6 weeks, at least 8 weeks, at least 10 weeks, at least 12 weeks, at least 24 weeks, at least 40 weeks, at least 60 weeks, at least 80 weeks, at least 100 weeks, at least 120 weeks, at least 6 weeks but not longer than 24 weeks, at least 12 weeks but not longer than 24 weeks, at least 6 weeks but not longer than 30 weeks, at least 12 weeks but not longer than 30 weeks, at least 6 weeks but not longer than 40 weeks, at least 12 weeks but not longer than 40 weeks, at least 6 weeks but not longer than 60 weeks, at least 12 weeks but not longer than 60 weeks, at least 6 weeks but not longer than 80 weeks, at least 12 weeks but not longer than 80 weeks, at least 6 weeks but not longer than 100 weeks, at least 12 weeks but not longer than 100 weeks, at least 6 weeks but not longer than 120 weeks, at least 12 weeks but not longer than 120 weeks, or at least 24 weeks but not longer than 120 weeks.

In one embodiment, the pharmaceutical composition provided by the present invention and containing an alkalinizing agent as an active ingredient may be evaluated for the treatment or preventive effect on the peripheral neuropathy induced by the chemotherapy drug at a point where administration of the pharmaceutical composition has been conducted continuously every day for two weeks from when the chemotherapy drug was administered.

### <Anticancer Pharmaceutical Composition with Suppressed Expression of Peripheral Neuropathy Induced by Cancer Chemotherapy Drug>

In one embodiment, the present invention is able to provide an anticancer pharmaceutical composition that exhibits suppressed expression of peripheral neuropathy induced by a chemotherapy drug, wherein this anticancer pharmaceutical composition contains an alkalinizing agent and a cancer chemotherapy drug as active ingredients.

As described above, it is known that some cancer chemotherapy drugs have a tendency to induce peripheral neuropathy.

Accordingly, by combining an alkalinizing agent with an anticancer drug that may induce peripheral neuropathy to form a combination drug, an anticancer pharmaceutical composition can be provided that exhibits suppressed expression of peripheral neuropathy induced by the cancer chemotherapy drug.

In one aspect, the expression "anticancer pharmaceutical composition that exhibits suppressed expression of peripheral neuropathy induced by a cancer chemotherapy drug" describes an anticancer pharmaceutical composition which, compared with an "anticancer pharmaceutical composition containing no alkalinizing agent" that contains the same amount of the anticancer drug as the pharmaceutical composition provided by the present invention, exhibits suppressed expression of peripheral neuropathy induced by the anticancer drug.

In another aspect, the "anticancer pharmaceutical composition that exhibits suppressed expression of peripheral neuropathy induced by a cancer chemotherapy drug" describes an anticancer pharmaceutical composition for which no expression of peripheral neuropathy induced by the anticancer chemotherapy drug develops.

Further, in yet another aspect, the "anticancer pharmaceutical composition that exhibits suppressed expression of peripheral neuropathy induced by a cancer chemotherapy drug" describes an anticancer pharmaceutical composition which, compared with an "anticancer pharmaceutical composition containing no alkalinizing agent" that contains the same amount of the anticancer drug as the pharmaceutical composition provided by the present invention, suppresses expression of peripheral neuropathy induced by the anticancer chemotherapy drug to 90% or less. This expression of peripheral neuropathy is preferably 85% or less, and more preferably 82% or less. In one aspect, the expression of peripheral neuropathy is within a range from 0 to 90%, from 0 to 85% in another aspect, from 0 to 82% in yet another aspect, from 20 to 85% in yet another aspect, from 20 to 82% in yet another aspect, from 30 to 82% in yet another aspect, from 40 to 82% in yet another aspect, and from 42 to 82% in yet another aspect. This rate of suppression of the expression of peripheral neuropathy can be calculated, for example, in the case of "pain", based on a suitable evaluation indicator such as the "pain-related score" described below.

In one embodiment, in the anticancer pharmaceutical composition provided by the present invention that exhibits suppressed expression of peripheral neuropathy induced by a cancer chemotherapy drug, examples of the chemotherapy drug (also referred to as the anticancer drug) that is included as an active ingredient include at least one anticancer drug selected from the group consisting of cytotoxic anticancer drugs such as paclitaxel, docetaxel, cabazitaxel, vinorelbine, vincristine, vinblastine, vindesine, eribulin, oxaliplatin, carboplatin, cisplatin and nelarabine; molecular targeted anticancer drugs such as bortezomib, ixazomib, romidepsin, gilteritinib, trastuzumab emtansine, brentuximab vedotin, obinutuzumab, blinatumomab, lorlatinib, entrectinib, encorafenib, binimetinib and pemigatinib; immunotherapy drugs such as nivolumab, ipilimumab, pembrolizumab, atezolizumab and avelumab; and other forms of anticancer drugs such as cytarabine, ifosfamide, nedaplatin, thalidomide, lenalidomide and pomalidomide, wherein at least one anticancer drug selected from the group consisting of paclitaxel, vincristine, oxaliplatin and bortezomib is preferred, and of these, paclitaxel or oxaliplatin is particularly preferred.

In one embodiment, in the anticancer pharmaceutical composition provided by the present invention that exhibits suppressed expression of peripheral neuropathy induced by a cancer chemotherapy drug, examples of the alkalinizing agent that is included as an active ingredient include the aforementioned pharmaceutically acceptable salts of citric acid or hydrates thereof, or mixtures thereof, and sodium bicarbonate.

In the anticancer pharmaceutical composition provided by the present invention that exhibits suppressed expression of peripheral neuropathy induced by a cancer chemotherapy drug, the blend ratio between the alkalinizing agent and the chemotherapy drug differs depending on the level of inducement of peripheral neuropathy caused by the included cancer chemotherapy drug, but in one embodiment, in the case where the alkalinizing agent is sodium citrate, potassium citrate, a hydrate thereof, or a mixture thereof, the amount of the alkalinizing agent per 1 part by mass of the anticancer drug may be within a range from 10 to 10,000 parts by mass, preferably from 20 to 6,000 parts by mass, and more preferably from 30 to 5,500 parts by mass.

In one embodiment, in the cases where the alkalinizing agent is sodium citrate, potassium citrate, a hydrate thereof, or a mixture thereof, and the anticancer drug is paclitaxel, the amount of the alkalinizing agent per 1 part by mass of the anticancer drug may be within a range from 10 to 150 parts by mass, preferably from 20 to 100 parts by mass, and more preferably from 30 to 90 parts by mass.

In one embodiment, in the cases where the alkalinizing agent is sodium citrate, potassium citrate, a hydrate thereof, or a mixture thereof, and the anticancer drug is oxaliplatin, the amount of the alkalinizing agent per 1 part by mass of the anticancer drug may be within a range from 10 to 150 parts by mass, preferably from 30 to 120 parts by mass, and more preferably from 50 to 85 parts by mass.

In one embodiment, in the cases where the alkalinizing agent is sodium citrate, potassium citrate, a hydrate thereof, or a mixture thereof, and the anticancer drug is vincristine, the amount of the alkalinizing agent per 1 part by mass of the anticancer drug may be within a range from 1,000 to 10,000 parts by mass, preferably from 3,000 to 7,000 parts by mass, and more preferably from 4,500 to 5,500 parts by mass.

In one embodiment, in the cases where the alkalinizing agent is sodium citrate, potassium citrate, a hydrate thereof, or a mixture thereof, and the anticancer drug is bortezomib, the amount of the alkalinizing agent per 1 part by mass of the anticancer drug may be within a range from 1,000 to 10,000 parts by mass, preferably from 3,000 to 7,000 parts by mass, and more preferably from 5,000 to 6,000 parts by mass.

In one embodiment, by administering the anticancer pharmaceutical composition provided by the present invention that exhibits suppressed expression of peripheral neuropathy induced by a cancer chemotherapy drug to a subject affected with cancer, for example, a human or other mammal, the cancer can be treated while suppressing the expression of peripheral neuropathy.

The cancer to be treated is determined by the type of anticancer drug included as an active ingredient in the anticancer pharmaceutical composition provided by the present invention. The anticancer drugs listed above are well-known anticancer drugs, and the cancers each anticancer drug is effective against are also known.

For example, compositions in which the active ingredient anticancer drug is paclitaxel are known to be effective against non-small cell lung cancer, breast cancer, ovarian cancer, uterine cancer, cervical cancer, stomach cancer, esophageal cancer, head and neck cancer, angiosarcoma, and germ cell tumors and the like. Compositions in which the active ingredient anticancer drug is oxaliplatin are known to be effective against colorectal cancer, stomach cancer, pancreatic cancer, and small intestine cancer. Compositions in which the active ingredient anticancer drug is vincristine are known to be effective against leukemia, malignant lymphoma, pediatric tumors, multiple myeloma, glioma, and pheochromocytoma. Further, compositions in which the active ingredient anticancer drug is bortezomib are known to be effective against multiple myeloma, mantle cell lymphoma, primary macroglobulinemia, and lymphoplasmacytic lymphoma.

In one embodiment, in the anticancer pharmaceutical composition provided by the present invention that exhibits suppressed expression of peripheral neuropathy induced by a cancer chemotherapy drug, the active ingredient anticancer drug may be effective against the subject cancer.

In one embodiment, the anticancer pharmaceutical composition provided by the present invention that exhibits suppressed expression of peripheral neuropathy induced by a cancer chemotherapy drug is administered orally or parenterally to a subject affected with cancer, for example, a human or other mammal, wherein examples of the parenteral administration include intravenous administration, subcutaneous administration, intramuscular administration, intra-articular administration, transmucosal administration, transdermal administration, transnasal administration, rectal administration, intrathecal administration, intraperitoneal administration, and local administration.

In one embodiment, in those cases where the active ingredient anticancer drug of the anticancer pharmaceutical composition provided by the present invention is itself a known anticancer drug, the anticancer drug of the pharmaceutical composition provided by the present invention may be administered to a subject requiring cancer treatment in accordance with established administration methods and dosage schedules.

By using the anticancer pharmaceutical composition provided by the present invention that exhibits suppressed expression of peripheral neuropathy induced by a cancer chemotherapy drug, the expression of peripheral neuropathy can be suppressed, and therefore even subjects for whom treatment with conventional anticancer drugs cannot be continued due to the expression of peripheral neuropathy can continue to be treated using the anticancer drug, and the QOL of those subjects can also be improved, meaning a more effective cancer treatment than conventional cancer treatments can be achieved.

In one embodiment, in those cases where the active ingredient anticancer drug of the anticancer pharmaceutical composition provided by the present invention is itself a known anticancer drug, the anticancer drug of the pharmaceutical composition provided by the present invention can be formulated in accordance with conventional formulations containing that anticancer drug.

In one embodiment, the formulation techniques described above in relation to the pharmaceutical composition containing an alkalinizing agent as an active ingredient can also be applied to the anticancer pharmaceutical composition provided by the present invention.

### 2. Combination Formulation

In one embodiment, the present invention provides a combination formulation for simultaneous, separate, or sequential administration in a cancer treatment, wherein the combination formulation includes at least two separate formulations composed of an alkalinizing agent and a cancer chemotherapy drug, and exhibits suppressed expression of peripheral neuropathy.

The alkalinizing agent and the cancer chemotherapy drug in the above combination formulation may employ the alkalinizing agent and cancer chemotherapy drug already disclosed above, and the formulation of the alkalinizing agent and the formulation of the cancer chemotherapy drug may also each employ any of the formulations already disclosed above.

The cancer to be treated by the combination formulation is determined by the type of chemotherapy drug (also referred to as an anticancer drug) included in the combination formulation provided by the present invention. The anticancer drugs listed above are well-known anticancer drugs, and the cancers each anticancer drug is effective against are also known.

In this description, the term "simultaneous" used in relation to the above combination formulation means the alkalinizing agent formulation and the cancer chemotherapy drug formulation contained in the combination formulation are administered at the same time in a timely manner.

In this description, the term "separate" used in relation to the above combination formulation means the alkalinizing agent formulation and the cancer chemotherapy drug formulation contained in the combination formulation are administered at different times during the course of a common treatment schedule.

In this description, the term "sequential" used in relation to the above combination formulation means one of either the alkalinizing agent formulation or the cancer chemotherapy drug formulation contained in the combination formulation is administered first and the other formulation is then administered thereafter, and may mean that one formulation is administered first and the other formulation administered immediately thereafter, that one formulation is administered first and the other formulation is then administered within the period during which the efficacy of the first formulation is still retained, or that one formulation is first administered repeatedly for a prescribed period, and the other formulation is then administered within the period during which the efficacy of the first formulation is still retained.

By administering the above combination formulation, a cancer treatment that exhibits suppressed expression of peripheral neuropathy induced by the cancer chemotherapy drug can be provided.

By using the combination formulation provided by the present invention, expression of peripheral neuropathy induced by the cancer chemotherapy drug can be suppressed, and therefore even subjects for whom treatment with conventional anticancer drugs cannot be continued due to the expression of peripheral neuropathy can continue to be treated using the anticancer drug, and the QOL of those subjects can also be improved, meaning a more effective cancer treatment than conventional cancer treatments can be achieved.

The ratio between the amounts of the alkalinizing agent and the cancer chemotherapy drug in the combination formulation provided by the present invention may be determined based on the ratio between the amounts of the alkalinizing agent and the cancer chemotherapy drug in the above anticancer pharmaceutical composition that exhibits suppressed expression of peripheral neuropathy induced by the cancer chemotherapy drug.

### 3. Pharmaceutical Kit

In one embodiment, the present invention can provide an anticancer pharmaceutical kit which contains an alkalinizing agent in a first compartment and a cancer chemotherapy drug in a second compartment, and exhibits suppressed expression of peripheral neuropathy.

The alkalinizing agent and the cancer chemotherapy drug in the pharmaceutical kit may use the alkalinizing agent and cancer chemotherapy drug already disclosed above. Further, the alkalinizing agent contained in the first compartment and the cancer chemotherapy drug contained in the second compartment have each preferably been produced as a formulation, with the formulation for the alkalinizing agent and the formulation for the cancer chemotherapy drug being as disclosed above.

The cancer to be treated by the pharmaceutical kit is determined by the type of cancer chemotherapy drug (also referred to as an anticancer drug) included in the pharmaceutical kit provided by the present invention. The anticancer drugs listed above are well-known anticancer drugs, and the cancers each anticancer drug is effective against are also known.

In one embodiment, the first compartment may be subdivided into two or three or more compartments in accordance with the number of doses and the dosage amount for the contained alkalinizing agent, with each compartment containing a sufficient amount of the alkalinizing agent for a single dose.

In one embodiment, the second compartment may be subdivided into two or three or more compartments in accordance with the number of doses and the dosage amount for the contained cancer chemotherapy drug, with each compartment containing a sufficient amount of the cancer chemotherapy drug for a single dose.

By using the pharmaceutical kit described above, a cancer treatment that exhibits suppressed expression of peripheral neuropathy induced by the cancer chemotherapy drug can be provided.

By using the pharmaceutical kit provided by the present invention, expression of peripheral neuropathy induced by the cancer chemotherapy drug can be suppressed, and therefore even subjects for whom treatment with conventional anticancer drugs cannot be continued due to the expression of peripheral neuropathy can continue to be treated using the anticancer drug, and the QOL of those subjects can also be improved, meaning a more effective cancer treatment than conventional cancer treatments can be achieved.

The ratio between the amounts of the alkalinizing agent and the cancer chemotherapy drug in the pharmaceutical kit provided by the present invention may be determined based on the ratio between the amounts of the alkalinizing agent and the cancer chemotherapy drug in the above anticancer pharmaceutical composition that exhibits suppressed expression of peripheral neuropathy induced by the cancer chemotherapy drug.

Examples of other embodiments of the present invention are described below.
<1-1> An alkalinizing agent or a pharmaceutical composition containing an alkalinizing agent, for use in treating or preventing peripheral neuropathy induced by a chemotherapy drug.
<1-2> The alkalinizing agent or pharmaceutical composition containing an alkalinizing agent for the use disclosed in <1-1>, wherein the peripheral neuropathy induced by a chemotherapy drug is pain, sensory disturbance, mobility impairment, autonomic neuropathy, or a complication thereof that is attributable to peripheral neuropathy.
<1-3> The alkalinizing agent or pharmaceutical composition containing an alkalinizing agent for the use disclosed in <1-1> or <1-2>, wherein the peripheral neuropathy induced by a chemotherapy drug is peripheral neuropathic pain.
<1-4> The alkalinizing agent or pharmaceutical composition containing an alkalinizing agent for the use disclosed in <1-3>, wherein the peripheral neuropathic pain is shooting pain, burning pain, pain (excluding shooting pain and burning pain), or tingling.
<1-5> The alkalinizing agent or pharmaceutical composition containing an alkalinizing agent for the use disclosed in <1-1> or <1-2>, wherein the peripheral neuropathy induced by a chemotherapy drug is a sensory disturbance attributable to peripheral neuropathy.
<1-6> The alkalinizing agent or pharmaceutical composition containing an alkalinizing agent for the use disclosed in <1-5>, wherein the sensory disturbance attributable to peripheral neuropathy is hypoesthesia, hyperesthesia or a sensory disorder.
<1-7> The alkalinizing agent or pharmaceutical composition containing an alkalinizing agent for the use disclosed in <1-6>, wherein the hyperesthesia is allodynia, and the sensory disorder is dysesthesia or paresthesia.
<1-8> The alkalinizing agent or pharmaceutical composition containing an alkalinizing agent for the use disclosed in <1-1> or <1-2>, wherein the peripheral neuropathy induced by a chemotherapy drug is autonomic neuropathy attributable to peripheral neuropathy.
<1-9> The alkalinizing agent or pharmaceutical composition containing an alkalinizing agent for the use disclosed in <1-8>, wherein a symptom caused by the autonomic neuropathy is urinary disturbance, sweating disorder, orthostatic hypotension, constipation, or paralytic ileus.
<1-10> The alkalinizing agent or pharmaceutical composition containing an alkalinizing agent for the use disclosed in any one of <1-1> to <1-9>, wherein the peripheral neuropathy induced by a chemotherapy drug is peripheral neuropathy having symptoms that manifest in the four limbs.
<1-11> The alkalinizing agent or pharmaceutical composition containing an alkalinizing agent for the use disclosed in any one of <1-1> to <1-10>, wherein the alkalinizing agent is a pharmaceutically acceptable salt of citric acid or a hydrate thereof, or a mixture thereof.
<1-12> The alkalinizing agent or pharmaceutical composition containing an alkalinizing agent for the use disclosed in any one of <1-1> to <1-11>, wherein the alkalinizing agent is sodium citrate, potassium citrate, a hydrate of sodium citrate or potassium citrate, or a mixture thereof.
<1-13> The alkalinizing agent or pharmaceutical composition containing an alkalinizing agent for the use disclosed in any one of <1-1> to <1-12>, wherein the alkalinizing agent contains a mixture of sodium citrate or a hydrate thereof, and potassium citrate or a hydrate thereof.
<1-14> The alkalinizing agent or pharmaceutical composition containing an alkalinizing agent for the use disclosed in any one of <1-1> to <1-13>, wherein the alkalinizing agent is sodium citrate or a hydrate thereof.
<1-15> The alkalinizing agent or pharmaceutical composition containing an alkalinizing agent for the use disclosed in any one of <1-1> to <1-14>, wherein the pharmaceutical composition is a tablet.
<1-16> The alkalinizing agent or pharmaceutical composition containing an alkalinizing agent for the use disclosed in any one of <1-1> to <1-10>, wherein the alkalinizing agent is sodium bicarbonate.
<1-17> The alkalinizing agent or pharmaceutical composition containing an alkalinizing agent for the use disclosed in any one of <1-1> to <1-16>, wherein the chemotherapy drug is a cancer chemotherapy drug.
<1-18> The alkalinizing agent or pharmaceutical composition containing an alkalinizing agent for the use disclosed in <1-17>, wherein the cancer chemotherapy drug is a cytotoxic anticancer drug, molecular targeted anticancer drug, immunotherapy drug, or other form of anticancer drug.
<1-19> The alkalinizing agent or pharmaceutical composition containing an alkalinizing agent for the use disclosed in <1-18>, wherein the cytotoxic anticancer drug is at least one anticancer drug selected from the group consisting of paclitaxel, docetaxel, cabazitaxel, vinorelbine, vincristine, vinblastine, vindesine, eribulin, oxaliplatin, carboplatin, cisplatin and nelarabine.
<1-20> The alkalinizing agent or pharmaceutical composition containing an alkalinizing agent for the use disclosed in <1-18>, wherein the molecular targeted anticancer drug is at least one anticancer drug selected from the group consisting of bortezomib, ixazomib, romidepsin, gilteritinib, trastuzumab emtansine, brentuximab vedotin, obinutuzumab, blinatumomab, lorlatinib, entrectinib, encorafenib, binimetinib and pemigatinib.
<1-21> The alkalinizing agent or pharmaceutical composition containing an alkalinizing agent for the use disclosed in <1-18>, wherein the immunotherapy drug is at least one anticancer drug selected from the group consisting of nivolumab, ipilimumab, pembrolizumab, atezolizumab and avelumab.
<1-22> The alkalinizing agent or pharmaceutical composition containing an alkalinizing agent for the use disclosed in <1-18>, wherein the other form of anticancer drug is at least one anticancer drug selected from the group consisting of cytarabine, ifosfamide, nedaplatin, thalidomide, lenalidomide and pomalidomide.
<1-23> The alkalinizing agent or pharmaceutical composition containing an alkalinizing agent for the use disclosed in <1-17>, wherein the cancer chemotherapy drug is at least one anticancer drug selected from the group consisting of paclitaxel, vincristine, oxaliplatin and bortezomib.
<1-24> The alkalinizing agent or pharmaceutical composition containing an alkalinizing agent for the use disclosed in <1-23>, wherein the cancer chemotherapy drug is paclitaxel or oxaliplatin.
<1-25> A combination formulation for use in a cancer treatment that exhibits suppressed expression of peripheral neuropathy, wherein
   the combination formulation includes at least two separate formulations composed of an alkalinizing agent and a cancer chemotherapy drug, for simultaneous, separate, or sequential administration.
<1-26> The combination formulation for the use disclosed in <1-25>, wherein the peripheral neuropathy is pain, sensory disturbance, mobility impairment, autonomic neuropathy, or a complication thereof that is attributable to peripheral neuropathy.
<1-27> The combination formulation for the use disclosed in <1-25> or <1-26>, wherein the peripheral neuropathy is peripheral neuropathic pain.
<1-28> The combination formulation for the use disclosed in <1-27>, wherein the peripheral neuropathic pain is shooting pain, burning pain, pain (excluding shooting pain and burning pain), or tingling.
<1-29> The combination formulation for the use disclosed in <1-25> or <1-26>, wherein the peripheral neuropathy is a sensory disturbance attributable to peripheral neuropathy.
<1-30> The combination formulation for the use disclosed in <1-29>, wherein the sensory disturbance attributable to peripheral neuropathy is hypoesthesia, hyperesthesia or a sensory disorder.
<1-31> The combination formulation for the use disclosed in <1-30>, wherein the hyperesthesia is allodynia, and the sensory disorder is dysesthesia or paresthesia.
<1-32> The combination formulation for the use disclosed in <1-25> or <1-26>, wherein the peripheral neuropathy is autonomic neuropathy attributable to peripheral neuropathy.
<1-33> The combination formulation for the use disclosed in <1-32>, wherein a symptom caused by the autonomic neuropathy is urinary disturbance, sweating disorder, orthostatic hypotension, constipation, or paralytic ileus.
<1-34> The combination formulation for the use disclosed in any one of <1-25> to <1-33>, wherein the peripheral neuropathy is peripheral neuropathy having symptoms that manifest in the four limbs.
<1-35> The combination formulation for the use disclosed in any one of <1-25> to <1-34>, wherein the alkalinizing agent is a pharmaceutically acceptable salt of citric acid or a hydrate thereof, or a mixture thereof.
<1-36> The combination formulation for the use disclosed in any one of <1-25> to <1-35>, wherein the alkalinizing agent is sodium citrate, potassium citrate, a hydrate of sodium citrate or potassium citrate, or a mixture thereof.
<1-37> The combination formulation for the use disclosed in any one of <1-25> to <1-36>, wherein the alkalinizing agent contains a mixture of sodium citrate or a hydrate thereof, and potassium citrate or a hydrate thereof.
<1-38> The combination formulation for the use disclosed in any one of <1-25> to <1-37>, wherein the alkalinizing agent is sodium citrate or a hydrate thereof.
<1-39> The combination formulation for the use disclosed in any one of <1-25> to <1-34>, wherein the alkalinizing agent is sodium bicarbonate.
<1-40> The combination formulation for the use disclosed in any one of <1-25> to <1-39>, wherein the cancer chemotherapy drug is a cytotoxic anticancer drug, molecular targeted anticancer drug, immunotherapy drug, or other form of anticancer drug.
<1-41> The combination formulation for the use disclosed in <1-40>, wherein the cytotoxic anticancer drug is at least one anticancer drug selected from the group consisting of paclitaxel, docetaxel, cabazitaxel, vinorelbine, vincristine, vinblastine, vindesine, eribulin, oxaliplatin, carboplatin, cisplatin and nelarabine.
<1-42> The combination formulation for the use disclosed in <1-40>, wherein the molecular targeted anticancer drug is at least one anticancer drug selected from the group consisting of bortezomib, ixazomib, romidepsin, gilteritinib, trastuzumab emtansine, brentuximab vedotin, obinutuzumab, blinatumomab, lorlatinib, entrectinib, encorafenib, binimetinib and pemigatinib.
<1-43> The combination formulation for the use disclosed in <1-40>, wherein the immunotherapy drug is at least one anticancer drug selected from the group consisting of nivolumab, ipilimumab, pembrolizumab, atezolizumab and avelumab.
<1-44> The combination formulation for the use disclosed in <1-40>, wherein the other form of anticancer drug is at least one anticancer drug selected from the group consisting of cytarabine, ifosfamide, nedaplatin, thalidomide, lenalidomide and pomalidomide.
<1-45> The combination formulation for the use disclosed in any one of <1-25> to <1-39>, wherein the cancer chemotherapy drug is at least one anticancer drug selected from the group consisting of paclitaxel, vincristine, oxaliplatin and bortezomib.
<1-46> The combination formulation for the use disclosed in <1-45>, wherein the cancer chemotherapy drug is paclitaxel or oxaliplatin.
<1-47> An anticancer pharmaceutical composition containing an alkalinizing agent and a cancer chemotherapy drug, for use in a cancer treatment that exhibits suppressed expression of peripheral neuropathy.
<1-48> The anticancer pharmaceutical composition for the use disclosed in <1-47>, wherein the peripheral neuropathy is pain, sensory disturbance, mobility impairment, autonomic neuropathy, or a complication thereof that is attributable to peripheral neuropathy.
<1-49> The anticancer pharmaceutical composition for the use disclosed in <1-47> or <1-48>, wherein the peripheral neuropathy is peripheral neuropathic pain.
<1-50> The anticancer pharmaceutical composition for the use disclosed in <1-49>, wherein the peripheral neuropathic pain is shooting pain, burning pain, pain (excluding shooting pain and burning pain), or tingling.
<1-51> The anticancer pharmaceutical composition for the use disclosed in <1-47> or <1-48>, wherein the peripheral neuropathy is a sensory disturbance attributable to peripheral neuropathy.
<1-52> The anticancer pharmaceutical composition for the use disclosed in <1-51>, wherein the sensory disturbance attributable to peripheral neuropathy is hypoesthesia, hyperesthesia or a sensory disorder.
<1-53> The anticancer pharmaceutical composition for the use disclosed in <1-52>, wherein the hyperesthesia is allodynia, and the sensory disorder is dysesthesia or paresthesia.
<1-54> The anticancer pharmaceutical composition for the use disclosed in <1-47> or <1-48>, wherein the peripheral neuropathy is autonomic neuropathy attributable to peripheral neuropathy.
<1-55> The anticancer pharmaceutical composition for the use disclosed in <1-54>, wherein a symptom caused by the autonomic neuropathy is urinary disturbance, sweating disorder, orthostatic hypotension, constipation, or paralytic ileus.
<1-56> The anticancer pharmaceutical composition for the use disclosed in any one of <1-47> to <1-55>, wherein the peripheral neuropathy is peripheral neuropathy having symptoms that manifest in the four limbs.
<1-57> The anticancer pharmaceutical composition for the use disclosed in any one of <1-47> to <1-56>, wherein the alkalinizing agent is a pharmaceutically acceptable salt of citric acid or a hydrate thereof, or a mixture thereof.
<1-58> The anticancer pharmaceutical composition for the use disclosed in any one of <1-47> to <1-57>, wherein the alkalinizing agent is sodium citrate, potassium citrate, a hydrate of sodium citrate or potassium citrate, or a mixture thereof.
<1-59> The anticancer pharmaceutical composition for the use disclosed in any one of <1-47> to <1-58>, wherein the alkalinizing agent contains a mixture of sodium citrate or a hydrate thereof, and potassium citrate or a hydrate thereof.
<1-60> The anticancer pharmaceutical composition for the use disclosed in any one of <1-47> to <1-59>, wherein the alkalinizing agent is sodium citrate or a hydrate thereof.
<1-61> The anticancer pharmaceutical composition for the use disclosed in any one of <1-47> to <1-56>, wherein the alkalinizing agent is sodium bicarbonate.
<1-62> The anticancer pharmaceutical composition for the use disclosed in any one of <1-47> to <1-61>, wherein the cancer chemotherapy drug is a cytotoxic anticancer drug, molecular targeted anticancer drug, immunotherapy drug, or other form of anticancer drug.
<1-63> The anticancer pharmaceutical composition for the use disclosed in <1-62>, wherein the cytotoxic anticancer drug is at least one anticancer drug selected from the group consisting of paclitaxel, docetaxel, cabazitaxel, vinorelbine, vincristine, vinblastine, vindesine, eribulin, oxaliplatin, carboplatin, cisplatin and nelarabine.
<1-64> The anticancer pharmaceutical composition for the use disclosed in <1-62>, wherein the molecular targeted anticancer drug is at least one anticancer drug selected from the group consisting of bortezomib, ixazomib, romidepsin, gilteritinib, trastuzumab emtansine, brentuximab vedotin, obinutuzumab, blinatumomab, lorlatinib, entrectinib, encorafenib, binimetinib and pemigatinib.
<1-65> The anticancer pharmaceutical composition for the use disclosed in <1-62>, wherein the immunotherapy drug is at least one anticancer drug selected from the group consisting of nivolumab, ipilimumab, pembrolizumab, atezolizumab and avelumab.
<1-66> The anticancer pharmaceutical composition for the use disclosed in <1-62>, wherein the other form of anticancer drug is at least one anticancer drug selected from the group consisting of cytarabine, ifosfamide, nedaplatin, thalidomide, lenalidomide and pomalidomide.
<1-67> The anticancer pharmaceutical composition for the use disclosed in any one of <1-47> to <1-61>, wherein the cancer chemotherapy drug is at least one anticancer drug selected from the group consisting of paclitaxel, vincristine, oxaliplatin and bortezomib.
<1-68> The anticancer pharmaceutical composition for the use disclosed in <1-67>, wherein the cancer chemotherapy drug is paclitaxel or oxaliplatin.
<1-69> A pharmaceutical kit for use in a cancer treatment that exhibits suppressed expression of peripheral neuropathy, wherein
   the pharmaceutical kit contains an alkalinizing agent in a first compartment and a cancer chemotherapy drug in a second compartment.
<1-70> The pharmaceutical kit for the use disclosed in <1-69>, wherein the peripheral neuropathy is pain, sensory disturbance, mobility impairment, autonomic neuropathy, or a complication thereof that is attributable to peripheral neuropathy.
<1-71> The pharmaceutical kit for the use disclosed in <1-69> or <1-70>, wherein the peripheral neuropathy is peripheral neuropathic pain.
<1-72> The pharmaceutical kit for the use disclosed in <1-71>, wherein the peripheral neuropathic pain is shooting pain, burning pain, pain (excluding shooting pain and burning pain), or tingling.
<1-73> The pharmaceutical kit for the use disclosed in <1-69> or <1-70>, wherein the peripheral neuropathy is a sensory disturbance attributable to peripheral neuropathy.
<1-74> The pharmaceutical kit for the use disclosed in <1-73>, wherein the sensory disturbance attributable to peripheral neuropathy is hypoesthesia, hyperesthesia or a sensory disorder.
<1-75> The pharmaceutical kit for the use disclosed in <1-74>, wherein the hyperesthesia is allodynia, and the sensory disorder is dysesthesia or paresthesia.
<1-76> The pharmaceutical kit for the use disclosed in <1-69> or <1-70>, wherein the peripheral neuropathy is autonomic neuropathy attributable to peripheral neuropathy.
<1-77> The pharmaceutical kit for the use disclosed in <1-76>, wherein a symptom caused by the autonomic neuropathy is urinary disturbance, sweating disorder, orthostatic hypotension, constipation, or paralytic ileus.
<1-78> The pharmaceutical kit for the use disclosed in any one of <1-69> to <1-77>, wherein the peripheral neuropathy is peripheral neuropathy having symptoms that manifest in the four limbs.
<1-79> The pharmaceutical kit for the use disclosed in any one of <1-69> to <1-78>, wherein the alkalinizing agent is a pharmaceutically acceptable salt of citric acid or a hydrate thereof, or a mixture thereof.
<1-80> The pharmaceutical kit for the use disclosed in any one of <1-69> to <1-79>, wherein the alkalinizing agent is sodium citrate, potassium citrate, a hydrate of sodium citrate or potassium citrate, or a mixture thereof.
<1-81> The pharmaceutical kit for the use disclosed in any one of <1-69> to <1-80>, wherein the alkalinizing agent contains a mixture of sodium citrate or a hydrate thereof, and potassium citrate or a hydrate thereof.
<1-82> The pharmaceutical kit for the use disclosed in any one of <1-69> to <1-81>, wherein the alkalinizing agent is sodium citrate or a hydrate thereof.
<1-83> The pharmaceutical kit for the use disclosed in any one of <1-69> to <1-78>, wherein the alkalinizing agent is sodium bicarbonate.
<1-84> The pharmaceutical kit for the use disclosed in any one of <1-69> to <1-83>, wherein the cancer chemotherapy drug is a cytotoxic anticancer drug, molecular targeted anticancer drug, immunotherapy drug, or other form of anticancer drug.
<1-85> The pharmaceutical kit for the use disclosed in <1-84>, wherein the cytotoxic anticancer drug is at least one anticancer drug selected from the group consisting of paclitaxel, docetaxel, cabazitaxel, vinorelbine, vincristine, vinblastine, vindesine, eribulin, oxaliplatin, carboplatin, cisplatin and nelarabine.
<1-86> The pharmaceutical kit for the use disclosed in <1-84>, wherein the molecular targeted anticancer drug is at least one anticancer drug selected from the group consisting of bortezomib, ixazomib, romidepsin, gilteritinib, trastuzumab emtansine, brentuximab vedotin, obinutuzumab, blinatumomab, lorlatinib, entrectinib, encorafenib, binimetinib and pemigatinib.
<1-87> The pharmaceutical kit for the use disclosed in <1-84>, wherein the immunotherapy drug is at least one anticancer drug selected from the group consisting of nivolumab, ipilimumab, pembrolizumab, atezolizumab and avelumab.
<1-88> The pharmaceutical kit for the use disclosed in <1-84>, wherein the other form of anticancer drug is at least one anticancer drug selected from the group consisting of cytarabine, ifosfamide, nedaplatin, thalidomide, lenalidomide and pomalidomide.
<1-89> The pharmaceutical kit for the use disclosed in any one of <1-69> to <1-83>, wherein the cancer chemotherapy drug is at least one anticancer drug selected from the group consisting of paclitaxel, vincristine, oxaliplatin and bortezomib.
<1-90> The pharmaceutical kit for the use disclosed in <1-89>, wherein the cancer chemotherapy drug is paclitaxel or oxaliplatin.
<2-1> A method for treating or preventing peripheral neuropathy induced by a chemotherapy drug, the method including administering an effective amount of an alkalinizing agent to a subject requiring treatment or prevention of peripheral neuropathy induced by the chemotherapy drug.
<2-2> A cancer treatment method that exhibits suppressed expression of peripheral neuropathy, the method including administering an effective amount of an alkalinizing agent and a cancer chemotherapy drug simultaneously, separately, or sequentially to a subject requiring cancer treatment.
<3-1> Use of an alkalinizing agent for producing a pharmaceutical composition for treating or preventing peripheral neuropathy induced by a chemotherapy drug.
<3-2> Use of an alkalinizing agent and a cancer chemotherapy drug for producing a combination formulation for simultaneous, separate, or sequential administration in a cancer treatment.

The use described above, wherein the combination formulation is a combination formulation that exhibits suppressed expression of peripheral neuropathy.
<3-3> Use of an alkalinizing agent and a cancer chemotherapy drug for producing an anticancer pharmaceutical composition that exhibits suppressed expression of peripheral neuropathy.
<3-4> Use of an alkalinizing agent and a cancer chemotherapy drug for producing an anticancer pharmaceutical kit that contains the alkalinizing agent in a first compartment and the cancer chemotherapy drug in a second compartment and exhibits suppressed expression of peripheral neuropathy.

### 4. Food Composition

In one embodiment, the present invention can provide an alkalinizing agent or a food composition containing an alkalinizing agent, for use in a non-therapeutic application that suppresses peripheral neuropathy induced by a chemotherapy drug.

The alkalinizing agents described above in the section entitled "1. Pharmaceutical Composition" may be used as the alkalinizing agent. Examples of the alkalinizing agent include pharmaceutically acceptable salts of citric acid (for example, alkali metal citrate salts or hydrates thereof, or a mixture thereof) or sodium bicarbonate, which may be used as food acceptable salts of citric acid, and a mixture of potassium citrate monohydrate (C₆H₅K₃O₇·H₂O) and sodium citrate dihydrate (C₆H₅Na₃O₇·2H₂O), or sodium citrate dihydrate is preferred.

The amount of the citric acid, pharmaceutically acceptable salt of citric acid or hydrate thereof, mixture thereof, or sodium bicarbonate in the food composition provided by the present invention may be determined appropriately depending on the type of food. Examples of the food composition include specific health foods, foods with functional claims, food for hospital patients, and supplements. There are no particular limitations on the form of these food compositions, provided the form contains an amount of the alkalinizing agent that is effective in achieving the above effects and can be ingested orally, and typical food and beverage forms are acceptable. Among the various formulations applicable to pharmaceutical compositions, formulations that are suited to oral administration such as tablets, capsules, and suspensions and the like may be used. The composition and production method for these formulations may employ the compositions and production methods described above for pharmaceutical formulations in the section entitled "1. Pharmaceutical Composition", or may apply known formulation techniques from the field of pharmaceutical formulation techniques.

For example, in the case of a specific health food, a food with functional claims, a food for hospital patients or a supplement, the food may contain 1/3 of a combined total of 1 to 3 g of potassium citrate monohydrate and sodium citrate dihydrate as active ingredients per serving of the food. For example, when the specific health food, food with functional claims, food for hospital patients or supplement is provided in the form of tablets, a single 300 mg to 600 mg tablet may contain 70 to 80% by weight of citric acid, a pharmaceutically acceptable salt of citric acid or a hydrate thereof, or a mixture thereof.

In those cases where the food composition provided by the present invention is not provided as a formulation, but is rather provided in the form of a typical food or beverage, then depending on the type of food or beverage, a person skilled in the art can produce the food or beverage in an appropriate manner, for example, by blending citric acid, a pharmaceutically acceptable salt of citric acid or a hydrate thereof, or a mixture thereof (for example, potassium citrate and/or sodium citrate), or sodium bicarbonate into the food raw material.

Examples of the form of the food or beverage include liquid, milky or paste-like foods such as beverages, soy sauce, milk, yoghurt and miso; semi-solid foods such as jellies or soft candies; solid foods such as candy, chewing gum, tofu and supplements; and powdered foods.

Examples of the beverages include fruit juices and fruit juice beverages, coffee beverages, oolong tea beverages, green tea beverages, tea beverages, barley tea beverages, vegetable beverages, carbonated beverages such as soft drinks, fruit juice extract-containing beverages, vegetable extract-containing beverages, flavored waters, sports beverages, and diet beverages.

The beverage may also contain one additive or a mixture of additives selected from among antioxidants, fragrances, various esters, organic acids, salts of organic acids, inorganic acids, salts of inorganic acids, inorganic salts, colorants, emulsifiers, preservatives, flavorings, sweeteners, acidifiers, fruit juice extracts, vegetable extracts, nectar extracts, pH regulators, and quality stabilizers.

The food composition provided by the present invention may be used in the same manner as the methods for using the pharmaceutical composition described above in the section entitled "1. Pharmaceutical Composition", or may also be used in applications not targeting the treatment or prevention of disease (also referred to as "non-therapeutic applications"). In other words, based on the alkalinizing agent contained in the food composition provided by the present invention, by using an amount of the alkalinizing agent in the food composition that is similar to the amount of the alkalinizing agent included in the above pharmaceutical composition, the food composition can be used for targeted subjects of the pharmaceutical composition. Further, in one embodiment, the "food composition" according to the present invention may be ingested as part of a non-therapeutic application for a subject undergoing administration of a chemotherapy drug, or to suppress the expression of peripheral neuropathy in a subject (for example, a human or other mammal) due to undergo administration of a chemotherapy drug, or to suppress peripheral neuropathy that has already manifested. In such cases, the therapeutic effect of the alkalinizing agent itself is basically the same regardless of whether the alkalinizing agent is a component of a pharmaceutical composition or a component of a food composition, and therefore the amount used of the food composition and the method used for administering the food composition may be adjusted appropriately on the basis of the alkalinizing agent in order to achieve the desired effect.

Food compositions which are used with subjects (such as humans or other mammals) that do not have a "pathological" or "abnormal" symptom, state or disease, namely, subjects (such as humans or other mammals) in a "healthy" or "normal" state, for the purposes of maintaining or extending that "healthy" or "normal" state, are sometimes referred to as "foods with functional claims".

The term "administration" described above in the section entitled "1. Pharmaceutical Composition" may also be applied to the "food composition" according to the present invention, but in the case of the "food composition" according to the present invention, the term "administration" may also be replaced with the term "ingestion". Accordingly, the terms "administer" or "administered" may be replaced with an expression such as "ingest", "ingested" or "be ingested" in accordance with the context.

The present invention is described below in further detail using a series of examples, but the present invention is in no way limited by these examples.

### Examples

### Example 1: Evaluation of Alkalinizing Agent in Oxaliplatin-Induced Mouse Neuropathic Pain Model

### (Test Substance)

For the alkalinizing agent, a mixture containing, per 1 g of dry weight, 463 mg of potassium citrate monohydrate (C₆H₅K₃O₇·H₂O), 390 mg of sodium citrate dihydrate (C₆H₅Na₃O₇·2H₂O) and 147 mg of pharmaceutically acceptable additives (Uralyt U Combination Powder, manufactured by Nippon Chemiphar Co., Ltd.) was used. Following weighing of the required amount of the Uralyt U combination powder (hereinafter also abbreviated as simply "Uralyt"), sufficient injection water was added to dissolve the powder and prepare a solution of the target concentration at the time of use.

In the Sham group and the control group, the injection water used as the solvent for the alkalinizing agent was used.

### (Animals)

Six-week old male C57BL/6J mice (from Japan SLC, Inc.) were used. The animals were provided with an approximately one-week acclimatization period, and only healthy individuals were then selected for testing.

### (Preparation of Test Animals)

Using the method described below, test animals: oxaliplatin (hereinafter also abbreviated as OXP) induced peripheral neuropathy model mice were prepared.

Grouping was conducted using the pain-related scores evaluated on the day prior to starting the test (the day prior to test substance administration) as a reference, and specifically, by dividing the individuals into each of the groups to ensure the same averaged pain-related score for each group. The three groups were composed of group 1: a Sham group, group 2: a control group, and group 3: an alkalinizing agent group.

For a period of 17 days starting from the day after grouping, the three groups were subjected to repeated oral administration (forced oral administration) twice per day, using injection water in the case of the Sham group and the control group, and a dosage of 1 g/kg of Uralyt in the case of the alkalinizing agent group. On the final day of administration, a single dose was administered for the day, so that a total 16.5 days' worth of dosages were administered across 17 days.

On day 3 of the injection water or Uralyt administration, 3 mg/kg of OXP was administered intraperitoneally in a single dose, thus preparing the test animals. Following weighing of the required amount, the OXP was added to and dissolved in a glucose injection solution (5%, manufactured by Otsuka Pharmaceutical Factory, Inc.) to generate a solution with a concentration of 0.3 mg/mL immediately prior to use. The Sham group was administered with the glucose injection solution (5%) used as the administration medium instead of the OXP solution.

### (Measurement Timings)

Following the OXP administration, the level of pain was evaluated over time. Pain evaluations were conducted by evaluating mechanical allodynia and cold dysesthesia. With the day of OXP administration taken as day 0, pain evaluations were conducted on days 0, 1, 3, 6, 8, 10 and 14, two hours after the administration of the injection water or Uralyt. A pain evaluation was also conducted on a day prior to the start of Uralyt administration, and this evaluation was recorded as the Pre value.

### (Pain Evaluations)

Each pain evaluation was conducted by transferring the individual to a mesh bottom individual cage (W110 × D180 × H150 mm) used for measuring the pain-related score, and then waiting 30 minutes for the individual to acclimatize to the new environment before conducting the evaluation.

Subsequently, in the case of a mechanical allodynia evaluation, the soles of the rear feet were stimulated with a von Frey hair (0.69 mN), and the response to the stimulus was scored.

In the case of a cold dysesthesia evaluation (only conducted for the OXP models), acetone was applied to the skin on the soles of the rear feet, and the aversive reaction following the acetone stimulus was scored.

### (Scoring Method)

### (1) Mechanical Allodynia

The von Frey hair was pressed perpendicularly onto the central portion of the sole of a rear foot, and held for 1 to 3 seconds in a position where the filament was slightly bent. Stimuli of the same strength were applied alternately to each rear foot at an interval of several seconds, and the average score after 3 repetitions on each foot, a total of six evaluations, was recorded as the score value (with a maximum score being 2).
0: no response, or sideways movement of rear feet
1: lifting of rear feet
2: flinching of rear feet, or licking of site of stimulus

### (2) Cold Dysesthesia

Acetone was applied to the skin on the sole of a rear foot, and following the response immediately following application (not evaluated), the response of the rear foot over the ensuring 10 seconds was observed. Acetone was applied alternately to each rear foot with a period of at least 20 seconds between applications, and the average score after 3 repetitions on each foot, a total of six evaluations, was recorded as the score value (with a maximum score being 2).
0: no response, or sideways movement of rear feet
1: lifting of rear feet
2: flinching of rear feet, or licking of site of stimulus

### (Results)

In the OXP-induced peripheral neuropathy model mice, the onset of cold dysesthesia which peaked on day 3 and mechanical allodynia which peaked on day 10 was confirmed (FIG. 1).

Repeated administration of the Uralyt U combination powder to the OXP-induced peripheral neuropathy model mice suppressed the onset of mechanical allodynia and cold dysesthesia (FIG. 2).

It is thought that the above results indicate that repeated oral administration of Uralyt U combination powder is extremely effective against OXP-induced peripheral neuropathy.

### Example 2: Evaluation of Alkalinizing Agent in Paclitaxel-Induced Mouse Neuropathic Pain Model

### (Test Substance)

The same test substance as Example 1 was used.

### (Animals)

The same type of animals as Example 1 were used. Specifically, six-week old male C57BL/6J mice (from Japan SLC, Inc.) were used. The animals were provided with an approximately one-week acclimatization period, and only healthy individuals were then selected for testing.

### (Preparation of Test Animals)

With the exceptions of using paclitaxel (hereinafter also abbreviated as PTX) as the chemotherapy drug that induces peripheral neuropathy, and conducting pain evaluations until day 18 from the point of PTX administration, the same method as Example 1 was used to administer 5 mg/kg of PTX intraperitoneally in a single dose, thus preparing test animals: paclitaxel-induced peripheral neuropathy model mice.

Following weighing of the required amount, the PTX was added to and dissolved in the following administration medium with a concentration of 0.5 mg/mL immediately prior to use.

The administration medium was prepared at the time of use by adding 10% (v/v) of each of Kolliphor and ethanol to a physiological saline solution.

### (Measurement Timings)

Following the PTX administration, the level of pain was evaluated over time. Pain evaluations were conducted by evaluating mechanical allodynia. With the day of PTX administration taken as day 0, pain evaluations were conducted on days 0, 1, 3, 5, 7, 10, 12, 14 and 18, two hours after the administration of the injection water or Uralyt. A pain evaluation was also conducted on a day prior to the start of Uralyt administration, and this evaluation was recorded as the Pre value.

### (Pain Evaluations)

Pain evaluations were conducted using the same method as Example 1.

### (Results)

In the PTX-induced peripheral neuropathy model mice, the onset of mechanical allodynia which peaked on day 14 was confirmed (FIG. 3).

Repeated administration of the Uralyt to the PTX-induced peripheral neuropathy model mice suppressed the onset of mechanical allodynia (FIG. 4).

It is thought that the above results indicate that repeated oral administration of Uralyt is extremely effective against PTX-induced peripheral neuropathy.

### Example 3: Evaluation of Alkalinizing Agent in Bortezomib-Induced Mouse Neuropathic Pain Model

### (Test Substance)

The same test substance as Example 1 was used.

### (Animals)

The same type of animals as Example 1 were used. Specifically, six-week old male C57BL/6J mice (from Japan SLC, Inc.) were used. The animals were provided with an approximately one-week acclimatization period, and only healthy individuals were then selected for testing.

### (Preparation of Test Animals)

With the exceptions of using bortezomib (hereinafter also abbreviated as BTZ) as the chemotherapy drug that induces peripheral neuropathy, and conducting pain evaluations until day 15 from the point of BTZ administration, the same method as Example 1 was used to administer 0.3 mg/kg of BTZ intravenously in a single dose, thus preparing test animals: bortezomib-induced peripheral neuropathy model mice.

Following weighing of the required amount, the BTZ was added to and dissolved in physiological saline solution with a concentration of 0.6 mg/mL immediately prior to use.

### (Measurement Timings)

Following the BTZ administration, the level of pain was evaluated over time. Pain evaluations were conducted by evaluating mechanical allodynia. With the day of BTZ administration taken as day 0, pain evaluations were conducted on days 0, 3, 6, 9, 12 and 15, two hours after the administration of the injection water or Uralyt. A pain evaluation was also conducted on a day prior to the start of Uralyt administration, and this evaluation was recorded as the Pre value.

### (Pain Evaluations)

Pain evaluations were conducted using the same method as Example 1.

### (Results)

In the BTZ-induced peripheral neuropathy model mice, the onset of mechanical allodynia which peaked on day 12 was confirmed (FIG. 5).

Repeated administration of the Uralyt to the BTZ-induced peripheral neuropathy model mice suppressed the onset of mechanical allodynia (FIG. 6).

It is thought that the above results indicate that repeated oral administration of Uralyt is extremely effective against BTZ-induced peripheral neuropathy.

### Example 4: Evaluation of Alkalinizing Agent in Vincristine-Induced Mouse Neuropathic Pain Model

### (Test Substance)

The same test substance as Example 1 was used.

### (Animals)

The same type of animals as Example 1 were used. Specifically, six-week old male C57BL/6J mice (from Japan SLC, Inc.) were used. The animals were provided with an approximately one-week acclimatization period, and only healthy individuals were then selected for testing.

### (Preparation of Test Animals)

With the exceptions of using vincristine (hereinafter also abbreviated as VCN) as the chemotherapy drug that induces peripheral neuropathy, and conducting pain evaluations until day 18 from the point of VCN administration, the same method as Example 1 was used to administer 0.1 mg/kg of VCN intraperitoneally in a single dose, thus preparing test animals: vincristine-induced peripheral neuropathy model mice.

Following weighing of the required amount, the VCN was added to and dissolved in physiological saline solution with a concentration of 0.01 mg/mL immediately prior to use.

### (Measurement Timings)

Following the VCN administration, the level of pain was evaluated over time. Pain evaluations were conducted by evaluating mechanical allodynia. With the day of VCN administration taken as day 0, pain evaluations were conducted on days 0, 3, 7, 10, 14 and 18, two hours after the administration of the injection water or Uralyt. A pain evaluation was also conducted on a day prior to the start of Uralyt administration, and this evaluation was recorded as the Pre value.

### (Pain Evaluations)

Pain evaluations were conducted using the same method as Example 1.

### (Results)

In the VCN-induced peripheral neuropathy model mice, the onset of mechanical allodynia which peaked on day 14 was confirmed (FIG. 7).

Repeated administration of the Uralyt to the VCN-induced peripheral neuropathy model mice suppressed the onset of mechanical allodynia (FIG. 8).

It is thought that the above results indicate that repeated oral administration of Uralyt is extremely effective against VCN-induced peripheral neuropathy.

### Example 5: Evaluation of Alkalinizing Agent in Paclitaxel-Induced Rat Neuropathic Pain Model

### (Test Substance)

The same test substance as Example 1 was used.

### (Animals)

Seven-week old male SD rats (from The Jackson Laboratory Japan, Inc.) were used. The animals were provided with an approximately one-week acclimatization period, and only healthy individuals were then selected for testing.

### (Preparation of Test Animals)

Using the method described below, test animals: PTX-induced peripheral neuropathy model rats were prepared.

Grouping was conducted using the 50% withdrawal threshold value evaluated on the day prior to starting the test (the day prior to administration of the test substance) as a reference, and specifically, by dividing the individuals into each of the groups to ensure the same averaged 50% withdrawal threshold value and averaged weight for each group. The three groups were composed of group 1: a Sham group, group 2: a control group, and group 3: an alkalinizing agent group.

For a period of 24 days starting from the day after grouping, the three groups were subjected to repeated oral administration (forced oral administration) twice per day, using injection water in the case of the Sham group and the control group, and a dosage of 1 g/kg of Uralyt in the case of the alkalinizing agent group. On the final day of administration, a single dose was administered for the day, so that a total 23.5 days' worth of dosages were administered across 24 days.

From day 3 of the injection water or Uralyt administration, 2 mg/kg of PTX was administered intraperitoneally on four alternate days (day 3, 5, 7 and 9 from the start of the injection water or Uralyt administration), thus preparing the test animals. The PTX was prepared at the time of use by adding 30 mg of Taxol injection solution to physiological saline solution to generate a solution with a concentration of 2 mg/mL.

The Sham group was administered with physiological saline solution instead of the PTX solution.

### (Measurement Timings)

Following the PTX administration, the level of pain was evaluated over time. Pain evaluations were conducted by evaluating mechanical allodynia. With the day on which PTX administration was started taken as day 0, pain evaluations were conducted on days 7, 14 and 21, three hours after the administration of the injection water or Uralyt. A pain evaluation was also conducted on the day prior to the start of Uralyt administration (day 3), and this evaluation was recorded as the Pre value.

### (Pain Evaluations)

Each pain evaluation was conducted by pressing a von Frey filament perpendicularly onto the central portion of the footpad on the sole of the left rear foot of the rat, and observing the avoidance response, with the 50% withdrawal threshold being calculated in accordance with the up-down method of Chaplan et al. (J. Neurosci. Methods. 1994; 53:55 to 63).

### (Results)

In the PTX-induced peripheral neuropathy model rats, the onset of mechanical allodynia which peaked on day 21 was confirmed (FIG. 9).

Repeated administration of Uralyt to the PTX-induced peripheral neuropathy model rats suppressed the onset of mechanical allodynia (FIG. 9).

It is thought that the above results indicate that repeated oral administration of Uralyt is extremely effective against peripheral neuropathy induced by repeated PTX administration.

### Example 6: Evaluation of Alkalinizing Agent in Paclitaxel-Induced Mouse Neuropathic Pain Model

In this example, the effects of alkalinizing agent administration on the spontaneous and mechanical stimulus-evoked firing of dorsal horn neurons in a paclitaxel-induced neuropathic pain model were confirmed.

### (Test Substance)

The same test substance as Example 1 was used.

### (Animals)

The same type of animals as Example 1 were used. Specifically, six-week old male C57BL/6J mice (from Japan SLC, Inc.) were used. The animals were provided with an approximately one-week acclimatization period, and only healthy individuals were then selected for testing.

### (Preparation of Test Animals)

With the exceptions of using paclitaxel as the chemotherapy drug that induces peripheral neuropathy, and conducting pain evaluations until day 14 from the point of PTX administration, the same method as Example 1 was used to administer 5 mg/kg of PTX intraperitoneally in a single dose, thus preparing test animals: paclitaxel-induced peripheral neuropathy model mice.

Following weighing of the required amount, the PTX was added to and dissolved in the following administration medium with a concentration of 0.5 mg/mL immediately prior to use.

The administration medium was prepared at the time of use by adding 10% (v/v) of each of Kolliphor and ethanol to a physiological saline solution.

### (Evaluations)

### (1) Evaluation of Pain due to Mechanical Allodynia

### (Measurement Timings)

Three days prior to, and then 13 days after PTX administration

### (Pain Evaluation)

Evaluations of the pain due to mechanical allodynia were conducted using the same method as Example 1.

### (Scoring Method)

Scoring was conducted using the same method as Example 1.

### (Results)

The onset of mechanical allodynia in the PTX-induced peripheral neuropathy model mice was confirmed on day 13, but the repeated administration of Uralyt suppressed the onset of mechanical allodynia (FIG. 10).

### (2) Electrophysiological Analysis

### (Preparation of Electrophysiology Test Animals)

Fourteen days after PTX administration, test animal mice were deeply anaesthetized with urethane (1.2 to 1.5 g/kg, i.p.), a thoracic spine level laminectomy was performed, and each mouse was set in a brain stereotaxis apparatus. Using a stereoscopic microscope, the dura mater, arachnoid mater and pia mater were peeled back sequentially to expose the dorsal root, and a space for insertion of an electrode was prepared. Subsequently, the site was saturated with 95% oxygen and 5% carbon dioxide, and the spinal cord surface was superfused with a Krebs solution that had been heated to 37°C at a rate of 10 to 15 mL/min. Then, a tungsten electrode (manufactured by FHC, Inc.) was inserted into the spinal cord dorsal horn cortex (10 to 150 µm (Laminae I-II)) at the L4-L5 insertion site and recordings were made.

### (Measurement Timings)

The prepared electrophysiology test animals were measured twice, before and after stimulation using a von Frey hair.

### (Electrophysiological Evaluation)

The signals obtained from the measurements were amplified using an EX1 (manufactured by Dagan Corporation), converted to digital data using a Digidata 1400A (manufactured by Molecular Devices, LLC), and then recorded using the software Clampex ver. 10.2 (from Molecular Devices, LLC). Analyses were conducted using the software Clamfit ver. 10.2 (from Molecular Devices, LLC). Firings were recorded for the state where no stimulus was applied to the mouse, and the firing frequency per unit of time was evaluated as the spontaneous firing. Subsequently, using an art paintbrush and hooked forceps, a single neuron was identified, the receptive field was also defined, and a 0.69 mN von Frey Filament (vFF) was pressed against this region for 5 seconds, the elicited firing was recorded as the mechanical stimulus-evoked firing, and the firing frequency per unit of time was evaluated.

### (Results)

Increases in the spontaneous firing frequency and the vFF-evoked firing frequency recorded at the spinal cord dorsal horn in the PTX-induced peripheral neuropathy model mice were both inhibited by repeated Uralyt administration (FIG. 11 and FIG. 12).

It is thought that the above results indicate that repeated oral administration of Uralyt is extremely effective against PTX-induced peripheral neuropathy.

### Example 7: Evaluation of Alkalinizing Agent in Oxaliplatin-Induced Mouse Neuropathic Pain Model

### (Test Substances)

For the alkalinizing agent, the same test substance as Example 1 and sodium bicarbonate (in this description also referred to as baking soda, manufactured by Tokyo Chemical Industry Co., Ltd., purity: at least 99.0%) were used. Following weighing of the required amount of the sodium bicarbonate, sufficient injection water was added to dissolve the sodium bicarbonate and prepare a solution of the target concentration at the time of use.

In the Sham group and the control group, the injection water used as the solvent for the alkalinizing agent was used.

### (Animals)

The same type of animals as Example 1 were used. Specifically, six-week old male C57BL/6J mice (from Japan SLC, Inc.) were used. The animals were provided with an approximately one-week acclimatization period, and only healthy individuals were then selected for testing.

### (Preparation of Test Animals)

Using the method described below, test animals: oxaliplatin-induced peripheral neuropathy model mice were prepared.

Grouping was conducted using the pain-related scores evaluated on the day prior to starting the test (the day prior to test substance administration) as a reference, and specifically, by dividing the individuals into each of the groups to ensure the same averaged pain-related score for each group. The four groups were composed of group 1: a Sham group, group 2: a control group, group 3: an alkalinizing agent (Uralyt) group, and group 4: an alkalinizing agent (sodium bicarbonate) group.

For a period of 13 days starting from the day after grouping, the four groups were subjected to repeated oral administration (forced oral administration) twice per day, using injection water in the case of the Sham group and the control group, a dosage of 1 g/kg of Uralyt in the case of the alkalinizing agent (Uralyt) group, and a dosage of 0.75 g/kg of sodium bicarbonate in the case of the alkalinizing agent (sodium bicarbonate) group. On the final day of administration, a single dose was administered for the day, so that a total 12.5 days' worth of dosages were administered across 13 days.

On day 3 of the injection water, Uralyt or sodium bicarbonate administration, 3 mg/kg of OXP was administered intraperitoneally in a single dose, thus preparing the test animals. Following weighing of the required amount, the OXP was added to and dissolved in a glucose injection solution (5%, manufactured by Otsuka Pharmaceutical Factory, Inc.) to generate a solution with a concentration of 0.3 mg/mL immediately prior to use.

The Sham group was administered with the glucose injection solution (5%) used as the administration medium instead of the OXP solution.

### (Measurement Timings)

Following the OXP administration, the level of pain was evaluated over time. Pain evaluations were conducted by evaluating mechanical allodynia. With the day of OXP administration taken as day 0, pain evaluations were conducted on days 0, 3, 5, 7 and 10, two hours after the administration of the injection water, Uralyt or sodium bicarbonate. A pain evaluation was also conducted on a day prior to the start of alkalinizing agent administration, and this evaluation was recorded as the Pre value.

### (Pain Evaluations)

The evaluations of mechanical allodynia were conducted using the same method as Example 1.

### (Results)

In the OXP-induced peripheral neuropathy model mice, the onset of mechanical allodynia which peaked on day 10 was confirmed (FIG. 13).

Repeated administration of the Uralyt U combination powder to the OXP-induced peripheral neuropathy model mice suppressed the onset of mechanical allodynia (FIG. 14).

Repeated administration of sodium bicarbonate to the OXP-induced peripheral neuropathy model mice suppressed the onset of mechanical allodynia, but the effect was smaller than that observed for the Uralyt administration group (FIG. 14).

It is thought that the above results indicate that repeated oral administration of an alkalinizing agent (and particularly Uralyt) is extremely effective against OXP-induced peripheral neuropathy.

### Example 8: Evaluation of Alkalinizing Agent in Paclitaxel-Induced Mouse Neuropathic Pain Model

### (Test Substances)

For the alkalinizing agent, the same test substance as Example 1 and sodium bicarbonate (in this description also referred to as baking soda, manufactured by Tokyo Chemical Industry Co., Ltd., purity: at least 99.0%) were used. Following weighing of the required amount of the sodium bicarbonate, sufficient injection water was added to dissolve the sodium bicarbonate and prepare a solution of the target concentration at the time of use.

In the Sham group and the control group, the injection water used as the solvent for the alkalinizing agent was used.

### (Animals)

The same type of animals as Example 1 were used. Specifically, six-week old male C57BL/6J mice (from Japan SLC, Inc.) were used. The animals were provided with an approximately one-week acclimatization period, and only healthy individuals were then selected for testing.

### (Preparation of Test Animals)

Using the method described below, test animals: paclitaxel-induced peripheral neuropathy model mice were prepared.

Grouping was conducted using the pain-related scores evaluated on the day prior to starting the test (the day prior to test substance administration) as a reference, and specifically, by dividing the individuals into each of the groups to ensure the same averaged pain-related score for each group. The four groups were composed of group 1: a Sham group, group 2: a control group, group 3: an alkalinizing agent (Uralyt) group, and group 4: an alkalinizing agent (sodium bicarbonate) group.

For a period of 17 days starting from the day after grouping, the four groups were subjected to repeated oral administration (forced oral administration) twice per day, using injection water in the case of the Sham group and the control group, a dosage of 1 g/kg of Uralyt in the case of the alkalinizing agent (Uralyt) group, and a dosage of 0.75 g/kg of sodium bicarbonate in the case of the alkalinizing agent (sodium bicarbonate) group. On the final day of administration, a single dose was administered for the day, so that a total 16.5 days' worth of dosages were administered across 17 days.

On day 3 of the injection water, Uralyt or sodium bicarbonate administration, 5 mg/kg of PTX was administered intraperitoneally in a single dose, thus preparing the test animals: paclitaxel-induced peripheral neuropathy model mice.

Following weighing of the required amount, the PTX was added to and dissolved in the following administration medium with a concentration of 0.5 mg/mL immediately prior to use.

The administration medium was prepared at the time of use by adding 10% (v/v) of each of Kolliphor and ethanol to a physiological saline solution.

### (Measurement Timings)

Following the PTX administration, the level of pain was evaluated over time. Pain evaluations were conducted by evaluating mechanical allodynia. With the day of PTX administration taken as day 0, pain evaluations were conducted on days 0, 1, 3, 5, 7, 10, 12 and 14, two hours after the administration of the injection water, Uralyt or sodium bicarbonate. A pain evaluation was also conducted on a day prior to the start of alkalinizing agent administration, and this evaluation was recorded as the Pre value.

### (Pain Evaluations)

The evaluations of mechanical allodynia were conducted using the same method as Example 1.

### (Results)

In the PTX-induced peripheral neuropathy model mice, the onset of mechanical allodynia which peaked on day 14 was confirmed (FIG. 15).

Repeated administration of Uralyt to the PTX-induced peripheral neuropathy model mice suppressed the onset of mechanical allodynia (FIG. 16).

Repeated administration of sodium bicarbonate to the PTX-induced peripheral neuropathy model mice suppressed the onset of mechanical allodynia, but the effect was smaller than that observed for the Uralyt administration group (FIG. 16).

It is thought that the above results indicate that repeated oral administration of an alkalinizing agent (and particularly Uralyt) is extremely effective against PTX-induced peripheral neuropathy.

### Industrial Applicability

The pharmaceutical composition, combination formulation and pharmaceutical kit provided by the present invention can treat or prevent peripheral neuropathy induced by chemotherapy drugs.

Further, by using the pharmaceutical composition, combination formulation and pharmaceutical kit provided by the present invention, a cancer treatment can be provided that exhibits suppressed expression of peripheral neuropathy induced by chemotherapy drugs, and particularly cancer chemotherapy drugs.

## Claims

1. A pharmaceutical composition for treating or preventing peripheral neuropathy induced by a chemotherapy drug, the composition comprising an alkalinizing agent.

2. The pharmaceutical composition according to Claim 1, wherein the peripheral neuropathy induced by a chemotherapy drug is pain, sensory disturbance, mobility impairment, autonomic neuropathy, or a complication thereof attributable to peripheral neuropathy.

3. The pharmaceutical composition according to Claim 1 or 2, wherein the peripheral neuropathy induced by a chemotherapy drug is peripheral neuropathic pain.

4. The pharmaceutical composition according to Claim 3, wherein the peripheral neuropathic pain is shooting pain, burning pain, pain (excluding shooting pain and burning pain), or tingling.

5. The pharmaceutical composition according to Claim 1 or 2, wherein the peripheral neuropathy induced by a chemotherapy drug is a sensory disturbance attributable to peripheral neuropathy.

6. The pharmaceutical composition according to Claim 5, wherein the sensory disturbance attributable to peripheral neuropathy is hypoesthesia, hyperesthesia or a sensory disorder.

7. The pharmaceutical composition according to Claim 6, wherein the hyperesthesia is allodynia, and the sensory disorder is dysesthesia or paresthesia.

8. The pharmaceutical composition according to Claim 1 or 2, wherein the peripheral neuropathy induced by a chemotherapy drug is autonomic neuropathy attributable to peripheral neuropathy.

9. The pharmaceutical composition according to Claim 8, wherein a symptom caused by the autonomic neuropathy is urinary disturbance, sweating disorder, orthostatic hypotension, constipation, or paralytic ileus.

10. The pharmaceutical composition according to any one of Claims 1 to 9, wherein the peripheral neuropathy induced by a chemotherapy drug is peripheral neuropathy having symptoms that manifest in the four limbs.

11. The pharmaceutical composition according to any one of Claims 1 to 10, wherein the alkalinizing agent is a pharmaceutically acceptable salt of citric acid or a hydrate thereof, or a mixture thereof.

12. The pharmaceutical composition according to any one of Claims 1 to 11, wherein the alkalinizing agent is sodium citrate, potassium citrate, a hydrate of sodium citrate or potassium citrate, or a mixture thereof.

13. The pharmaceutical composition according to any one of Claims 1 to 12, wherein the alkalinizing agent comprises a mixture of sodium citrate or a hydrate thereof, and potassium citrate or a hydrate thereof.

14. The pharmaceutical composition according to any one of Claims 1 to 13, wherein the alkalinizing agent is sodium citrate or a hydrate thereof.

15. The pharmaceutical composition according to any one of Claims 1 to 14, wherein the pharmaceutical composition is a tablet.

16. The pharmaceutical composition according to any one of Claims 1 to 10, wherein the alkalinizing agent is sodium bicarbonate.

17. The pharmaceutical composition according to any one of Claims 1 to 16, wherein the chemotherapy drug is a cancer chemotherapy drug.

18. The pharmaceutical composition according to Claim 17, wherein the cancer chemotherapy drug is a cytotoxic anticancer drug, molecular targeted anticancer drug, immunotherapy drug, or other form of anticancer drug.

19. The pharmaceutical composition according to Claim 18, wherein the cytotoxic anticancer drug is at least one anticancer drug selected from the group consisting of paclitaxel, docetaxel, cabazitaxel, vinorelbine, vincristine, vinblastine, vindesine, eribulin, oxaliplatin, carboplatin, cisplatin and nelarabine.

20. The pharmaceutical composition according to Claim 18, wherein the molecular targeted anticancer drug is at least one anticancer drug selected from the group consisting of bortezomib, ixazomib, romidepsin, gilteritinib, trastuzumab emtansine, brentuximab vedotin, obinutuzumab, blinatumomab, lorlatinib, entrectinib, encorafenib, binimetinib and pemigatinib.

21. The pharmaceutical composition according to Claim 18, wherein the immunotherapy drug is at least one anticancer drug selected from the group consisting of nivolumab, ipilimumab, pembrolizumab, atezolizumab and avelumab.

22. The pharmaceutical composition according to Claim 18, wherein the other form of anticancer drug is at least one anticancer drug selected from the group consisting of cytarabine, ifosfamide, nedaplatin, thalidomide, lenalidomide and pomalidomide.

23. The pharmaceutical composition according to Claim 17, wherein the cancer chemotherapy drug is at least one anticancer drug selected from the group consisting of paclitaxel, vincristine, oxaliplatin and bortezomib.

24. The pharmaceutical composition according to Claim 23, wherein the cancer chemotherapy drug is paclitaxel or oxaliplatin.

25. A combination formulation for simultaneous, separate, or sequential administration in a cancer treatment, wherein
the combination formulation comprises at least two separate formulations composed of an alkalinizing agent and a cancer chemotherapy drug, and exhibits suppressed expression of peripheral neuropathy.

26. An anticancer pharmaceutical composition that exhibits suppressed expression of peripheral neuropathy, the composition comprising an alkalinizing agent and a cancer chemotherapy drug.

27. An anticancer pharmaceutical kit comprising an alkalinizing agent in a first compartment and a cancer chemotherapy drug in a second compartment, wherein the pharmaceutical kit exhibits suppressed expression of peripheral neuropathy.
